(19)

Europäisches
Patentamt
European
Patent Office
Office européen
des brevets

(11) **EP 4 652 948 A1**

(12) **EUROPEAN PATENT APPLICATION**

(43) Date of publication:
**26.11.2025 Bulletin 2025/48**

(51) International Patent Classification (IPC):
**A61B 18/20** (2006.01)  **A61F 9/008** (2006.01)
**A61N 5/06** (2006.01)  **A61N 7/00** (2006.01)
**A61B 34/30** (2016.01)  **A61N 5/10** (2006.01)

(21) Application number: **24178004.8**

(22) Date of filing: **24.05.2024**

(52) Cooperative Patent Classification (CPC):
**A61B 18/203;** A61B 18/201; A61B 2018/00452;
A61B 2018/00642; A61B 2018/00904;
A61B 2018/20359

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Fotona d.o.o.**
**1000 Ljubljana (SI)**

(72) Inventors:
• **VRABIC, Rok**
**1000 Ljubljana (SI)**
• **JEZERSEK, Matija**
**1235 Radomije (SI)**
• **LUKAC, Matjaz**
**1000 Ljubljana (SI)**

• **LUKAC, Nejc**
**1000 Ljubljana (SI)**
• **KOSIR, Jure**
**1230 Domzale (SI)**
• **SENEGACNIK, Matej**
**1000 Ljubljana (SI)**
• **GAJSEK, Rok**
**1356 Dobrova (SI)**
• **MAROLT, Bor**
**1351 Brezovica pri Ljubljani (SI)**
• **KRUSIC, Spela**
**1262 Dol pri Ljubljani (SI)**
• **JUVAN, Vid**
**3320 Velenje (SI)**

(74) Representative: **Bardehle Pagenberg
Partnerschaft mbB
Patentanwälte Rechtsanwälte
Prinzregentenplatz 7
81675 München (DE)**

(54) **APPARATUS FOR TREATMENT OF A TARGET AREA**

(57)    The present invention relates to an apparatus for treatment of a target area of a patient by an energy beam. The apparatus comprises means for receiving at least one 3D image of the target area; means for delivering the energy beam to at least a portion of the target area and means for automatically moving the energy beam, preferably by moving at least a portion of the means for delivering the energy beam, such that energy is delivered essentially uniformly across the target area. In particular, the apparatus is adapted to control, during the moving of the energy beam, an effective distance of the means for delivering the energy beam and/or an orientation of the energy beam relative to the target area, at least in part based on the at least one 3D image of the target area.

**Fig. 1A**

**Description**

Field of the invention

The present invention relates to an apparatus for treatment of a target area, in particular wherein the treatment comprises automatically delivering energy to surfaces of human or animal tissues. The present invention also relates to a control method and a computer program usable for such treatment.

Technical background

In medical applications where energy is delivered to tissue for heating, coagulation or destruction of targets, a non-specific heating of the tissue, e.g., the epidermis, is a common side effect. Energy-based medical devices typically used for heating, coagulation or destruction include, for example, a laser, an intense pulse light (IPL) device or a radio frequency (rf) device.

In treatments using energy-based medical devices, a certain amount of energy needs to be delivered to achieve the desired effect on the target chromophore or target structure. For example, in applications for hair removal by means of a laser, enough laser fluence (energy/area) needs to be delivered to the hair bulb to achieve its destruction, but, at the same time, epidermal damage should be avoided or at least minimized. As many laser wavelengths, especially the ones which penetrate the skin surface, are absorbed in melanin (which is abundantly present in the epidermis), heating of the epidermis is an inevitable side effect of many laser treatments. Often, the threshold fluence which is needed for destroying the target chromophore or structure is very close to the fluence threshold for epidermal injury. Besides, uncontrolled heating of the epidermis above its coagulation temperature of 65 to 70°C for a prolonged period of time can induce acute epidermal damage or blistering and can also lead to scarring and hypopigmentation. To avoid these complications, while at the same time allowing enough energy to be delivered to the target structure, cooling of the epidermal surface layer may become necessary.

Exemplary applications where high energies are used such that cooling is necessary for avoiding epidermal damage include not only laser hair removal, but also coagulation of veins and vascular lesions.

In other applications, e.g., non-invasive fat reduction, relatively low energy densities are delivered during a prolonged exposure to an energy-based medical device (e.g., a laser, a radio frequency device, or another energy source) which leads to a prolonged heating of subdermal fat to temperatures above 42° C. This causes an apoptosis of adipose cells and a reduction of the fat layer. In these methods, epidermal cooling may be used for avoiding a prolonged heating of the epidermis, thus maximizing patient safety and comfort.

For treatment of a target area, e.g., a portion of human tissue subject to hair removal, involving the delivery of energy by an energy-based medical device to at least a portion of the tissue and cooling by a cooling system of at least a portion of the tissue, the energy-based medical device and the cooling system have previously been combined into a single, handheld device. Such a combined handheld device, also called handheld laser epilator, typically relies on an IPL device which is configured to irradiate an area of a predefined size. This predefined size is typically adjusted by embedding the IPL device in a housing, which has a flash/contact window acting as a light-transmissive section whose extent corresponds to the predefined size. Because the handheld device should be handy, the predefined size is typically much smaller than the size of the treatment area, which makes it necessary to manually move the handheld device, and thus the light-transmissive section, across the target area.

However, if such handheld device is applied manually, in self-treatment or by another person, the device is not moved uniformly across the target area due to imprecise movement and/or coordination of arm and hand. In particular, there will generally be a halt when the movement includes a change in direction. The IPL device is not aware of that halt and continues to irradiate a portion of the treatment area through the flash window. This leads to an overtreatment in the edges of the target area and thus to an overexposure. Further, as the handheld device may be used in direct contact with the skin or tissue, the resulting friction prevents a uniform movement across the target area. In particular, as the treatment leads to an increase of skin or tissue temperature, this may result in a secretion of transpiration, rendering a precise movement of the handheld device impossible. This increases the chances of burns and other adverse reactions, including skin irritation, redness, scars and swelling. In addition, during the treatment, the user might forget which region has already been treated by the device and/or might enlarge and/or might restrict the target area, which leads to a substantial overtreatment (or undertreatment).

Furthermore, where needed, the direct contact between the tissue and the device, in particular the flash window, may result in the formation of debris, i.e., dermatologic tissue accumulates at the flash window as treatment laser pulses are delivered. Therefore, the flash window must be cleaned regularly to keep the flash/contact window and/or the whole device from local, intense overheating, which thermally and mechanically stresses the flash/contact window.

If an additional cooling system is integrated into the handheld device, the cooling system is configured to cool the area to which the flash window is pointing. This is typically achieved by adapting the cooling system within the handheld device or housing such that it also operates through the flash window. However, such a construction only allows for cooling the area of tissue corresponding to the flash window and thus only a presently irradiated area. Further, the amount of cooling depends on how the handheld device is moved across the target area. More precisely, if the device is moved slowly, the cooling device can cool a portion of the target area for a longer time, increasing the amount of cooling, but if the device moves fast, the amount of cooling can be substantially decreased. This results in a non-uniform cooling profile of the target area which may contribute to overtreatment and overexposure (or undertreatment and underexposure).

One implementation of a laser tissue treatment device is described in US 6,273,885 B1. It comprises a laser which emits energy and a device for surface cooling of tissue such that the energy is directed through said cooling device in contact with tissue.

US 2007/0208326 A1 describes a hair removal device including a cooling surface which is used in contact with skin prior to exposure to hair tissue-damaging laser light passing from a radiation source through a recessed window.

In view of the above, there is a need to address at least some of the above-described disadvantages of the prior art.

## Summary of the invention

A first aspect relates to an apparatus for treatment of a target area of a patient by an energy beam. The apparatus comprises means for receiving at least one 3D image of the target area and means for delivering the energy beam to at least a portion of the target area. The apparatus further comprises means for automatically moving the energy beam, preferably by moving at least a portion of the means for delivering the energy beam, such that energy is delivered essentially uniformly across the target area. In addition, the apparatus is adapted to control, during the moving of the energy beam, an effective distance of the means for delivering the energy beam relative to the target area, at least in part based on the at least one 3D image of the target area. Alternatively, or in addition, the apparatus is adapted to control, during the moving of the energy beam an orientation of the energy beam relative to the target area, at least in part based on the at least one 3D image of the target area.

A target area of a patient may comprise a portion of a surface of a body of the patient. For example, the target area may comprise tissue of the patient. Specifically, the surface may comprise an outer body surface of a human body covered by skin, the surface of internal organs covered by mucosa and/or the tissue surface being exposed during an open surgery or because of an injury. For example, the target area may be a predefined area, e.g., an area of the patient specified before the treatment. In addition, or alternatively, the treatment area may be a variable area, e.g., the treatment area may be changed or adapted during the treatment.

In general, the treatment of the target area of the patient may comprise covering surface procedures, e.g., dermatological and/or aesthetic procedures. For example, the treatment may comprise at least one of hair removal, skin toning, skin whitening and/or treating pigmented lesions. Some of these applications may be non-therapeutic but purely cosmetic. In particular, the treatment may comprise tattoo treatments, vascular treatments, skin resurfacing, scar reduction, skin tightening, skin rejuvenation, intense heat shock biomodulation, transdermal lipolysis, low level laser therapy, low level laser therapy and/or hair growth stimulation. Alternatively, or in addition, the treatment may comprise mucosa covering procedures including vulva whitening and snoring treatments as well as exposed tissue surface procedures include tissue debridement and ablation, e.g., coagulation and/or and disinfection.

In some embodiments, the target area may be a connected area, e.g., the target area cannot be decomposed into at least two disjoint target areas or two non-adjacent target areas. Generally, the target area may comprise a convex area, e.g., the target area may comprise a convex hull of a set of boundary points. For example, the boundary of the target area may comprise a curved boundary. In addition, or alternatively, the boundary may comprise line segments. In particular, the target area may comprise an area that is the convex hull of a finite number of points, e.g., the target area may comprise a convex polygon.

Generally, the target area may be larger than a diameter of the energy beam. A diameter of the energy beam may be associated with the smallest possible portion of the target area which receives (at least) 50%, preferably (at least) 70%, more preferably (at least) 90%, even more preferably (at least) 95%, most preferably (at least) 99% or even 100% of the energy delivered by the energy beam at a given point in time. The beam diameter may comprise a circular geometry and/or an elliptical geometry. For example, if the beam comprises a circular geometry, the diameter may be specified by a single parameter. If the beam comprises an elliptical geometry, the diameter may be specified by a major and/or a minor axis of the elliptical geometry. In addition, or alternatively, the diameter of the beam may be associated with a full width at half maximum of the beam and/or a $1/e^2$ width of the beam. In particular, the energy beam may comprise a Gaussian beam. A Gaussian beam may be a beam whose amplitude envelope in a transverse plane with respect to the treatment area is given by a Gaussian function.

In some embodiments, the energy beam and/or the energy distribution of the energy beam may not be homogeneous across the beam. For example, the energy beam may comprise a patterned and/or fractional beam. A patterned beam may be an energy beam wherein the beam can be decomposed into subbeams. A subbeam may be an area of the beam wherein the energy distribution is higher than a certain threshold. In other words, subbeams are spatially separated by areas, wherein the energy distribution of the separating area is lower than a threshold. The geometry of at least one subbeam may correspond to the geometry of the beam. In addition, or alternatively, the geometry of at least one subbeam may differ from the geometry of the energy beam. In particular, the energy beam may comprise a circular geometry, while at least one subbeam may comprise an elliptical and/or polygonal geometry, e.g., a pentagon, a hexagon, a rectangle and/or a triangle. For example, the patterned beam may comprise a honeycomb pattern.

The apparatus comprises means for receiving at least one 3D image of the target area. In other words, the means for receiving the 3D image is configured to receive a 3D image of the target area. In general, the 3D image of the target area may be a 3D image of an area comprising the target area. The 3D image of the target area may be an image of the target area which comprises information about the surface structure of the target area. In particular, the 3D image of the target area may comprise information about a height and/or depth profile of the target area. Generally, the 3D image of the target area may associate to at least one point of the target area a height and/or depth. Preferably, the 3D image associates to any point of the target area a height and/or depth. For example, a height and/or depth of a point of the target area may refer to a distance of the point with respect to a reference point and/or reference plane. In some embodiments, the 3D image of the target area may associate to at least a part of the target area a height and/or depth. In particular, a size and/or geometry of the part may depend on a resolution of the means for receiving the 3D image.

Furthermore, the means for receiving at least one 3D image of the target area may receive more than one 3D image of the target area. For example, the means for receiving the at least one 3D image may receive 3D images of the target area from different perspectives. In particular, the means for receiving the at least one 3D image may receive a first 3D image of the target area from a first perspective and a second 3D image of the target area from a second perspective. In addition, or alternatively, the means for receiving the at least one 3D image may receive 3D images comprising differ parts of the target area. For example, the means for receiving the at least one 3D image may receive a first 3D image of a first part of the target area and a second 3D image of a second part of the target area. Preferably, the first part and the second part of the target area comprise the target area, e.g., the first part and the second part cover the target area.

Specifically, the means for receiving the 3D image may comprise a camera, preferably a depth-sensing camera. For example, the camera may capture a 3D image of the target area. In some embodiments, the means for receiving the 3D image may be at least partially attached to the means for delivering the energy beam and/or the means for automatically moving.

In addition, or alternatively, the means for receiving a 3D image may comprise an interface. For example, the interface may be configured to receive data associated with the 3D image of the target area. In particular, the means for receiving the 3D image may be configured to receive the 3D image from a remote device. In some embodiments, the remote device may not be attached to the means for delivering the energy beam and/or the means for automatically moving. The remote device may be a camera, preferably a depth-sensing camera. For example, the means for receiving the 3D image may be configured to receive the 3D image from a camera over a channel, e.g., a wired and/or wireless channel.

The apparatus further comprises means for automatically moving the energy beam. Specifically, the means for automatically moving may move the energy beam such that energy is delivered essentially uniformly across the target area. In particular, the means for automatically moving may move the energy beam across the target area such that energy is delivered essentially uniformly across the target area.

Delivering energy essentially uniformly across the target area may comprise that a distribution of energy delivered to the target area is essentially uniform across the target area. For example, the distribution of energy delivered to the target area may be essentially a flat and/or constant and/or homogeneous distribution across the target area. Particularly, an essentially unform distribution of energy delivered to the target area may comprise that the energy distribution across the target area may deviate from a predefined value by not more than 50%, preferably not more than 30%, more preferably not more than 20%, even more preferably not more than 10%, most preferably not more than 10%.

Automatically moving the energy beam may comprise moving at least a portion of the means for delivering the energy beam. For example, moving at least a portion of the means for delivering the energy beam may comprise to move the means for delivering the energy beam across the target area. In particular, moving the energy beam such that energy is delivered essentially uniformly across the target area may comprise moving at least a portion of the means for delivering energy essentially uniformly across the target area.

Furthermore, the apparatus may be adapted to control, during the moving of the energy beam, an effective distance of the means for delivering the energy beam relative to the target area. The control of the effective distance may be at least in part based on the at least one 3D image of the target area. Specifically, the control of the effective distance may comprise a control of a spatial distance of the means for delivering the energy to the target area. In addition, or alternatively, the control of the effective distance may comprise a control of a focus of the means for delivering the energy beam.

In general, the control of the effective distance may be based at least in part on the at least one 3D image of the target area. The effective distance may be a distance with respect to a portion of the target area, e.g., for different portions of the target area, the effective distance may be computed separately. For example, the control of the effective distance may be based on a height and/or depth profile associated with the at least one 3D image.

In some embodiments, the control of the effective distance may comprise a control of a spatial distance to the target area. For example, a spatial distance to the target area may comprise a distance of the means for delivering the energy beam to the target area. In particular, the spatial distance to the target area may comprise a distance of the means for delivering the energy beam to at least a portion of the target area. For example, the at least portion of the target area may be associated with a portion of the target area to which the energy beam is delivered. In general, the control of the effective distance may comprise that the means for automatically moving the energy beam moves the means for delivering the energy beam.

In addition, or alternatively, the apparatus may be adapted to control, during the moving of the energy beam, an orientation of the energy beam relative to the target area. The control of the effective distance may be at least in part based on the at least one 3D image of the target area. Specifically, the control of the orientation may comprise a control of an orientation of at least a portion of the means for delivering the energy beam relative to the at least portion of the target area.

The orientation of the energy beam relative to the target area may comprise an orientation of the energy beam with respect to a portion of the target area. For example, the portion of the target area, e.g., a size and/or a geometry of the portion, may be associated with the size and/or geometry of the energy beam. Preferably, the size and/or the geometry of the portion may be equal to the size and/or geometry of the energy beam. The orientation of the energy beam with respect to the portion of the target area may comprise an orientation of a direction of arrive of the energy beam and the portion of the target area. For example, the direction of arrival of the energy beam may be associated with an angle with respect to the portion, e.g., an incident angle. In particular, the angle may depend on how the energy beam impinges the portion. The angle with respect to the portion may be an angle between the direction of arrival of the energy beam and a normal vector associated with the portion of the target area. In particular, the normal vector associated with the portion of the target area may be a normal vector based on the at least one 3D image. For example, the normal vector associated with the portion of the target area may comprise a vector which is normal to a plane associated with the portion of the target area. The plane associated with the portion of the target area may comprise a plane which approximates the portion. For example, the plane may be plane such that a distance between the plane and the portion of the target area is minimized. The plane may be an average of planes which are tangent to at least a point of the target area.

In general, the control of the orientation of the energy beam relative to the target area may comprise to control an orientation of at least a portion of the means for delivering the energy beam. For example, the control of the orientation of at least a portion of the means for delivering the energy beam may comprise to control a spatially orientation of at least a portion if the means for delivering the energy beam. In particular, the control may comprise to control the orientation of the means for delivering the energy beam. For example, the control of the orientation may comprise moving the means for delivering the energy beam relative to the target area. In addition, or alternatively, the control of the orientation of at least a portion of the means for delivering the energy beam may comprise to move optical elements of the means for delivering the energy beam and/or to control a current associated with the means for delivering the energy.

Furthermore, the apparatus may be configured to, during the moving of the energy beam, maintain an essentially perpendicular orientation relative to the target area.

In general, the perpendicular orientation relative to the target area may comprise a perpendicular orientation relative to a portion of the target area. For example, the perpendicular orientation may comprise that the orientation of the energy beam may deviate from the normal vector associated with the portion of the target area by at most 20°, preferably at most 15°, more preferably at most 10°, even more preferably at most 5°, most preferably at most 2°. In other words, the direction of arrival of the energy beam and the normal vector of the portion define an angel of at most 20°, preferably at most 15°, more preferably at most 10°, even more preferably at most 5°, most preferably at most 2°.

Moving the energy beam such as to maintain an essentially perpendicular orientation relative to the target area energy contributes to the essentially uniformly delivering of energy across the target area. For example, deviating from an essentially perpendicular orientation relative to the target area may result in an inhomogeneous energy distribution of the energy beam. In particular, a different amount of energy may be delivered to different parts of the target area. Therefore, maintaining an essentially perpendicular orientation relative to the target area minimizes the risk of over- and/or undertreatment of parts of the target area, thus maximizes the safety and well-being of the patient and thereby optimized the quality of the treatment.

It is noted that the aspects described herein are not limited to delivering energy essentially uniformly across the target area. They may also be used in general for non-uniform treatments, if so desired. For example, the means for automatically moving may be configured to apply a certain, predetermined fluence locally that may vary across the target area.

In general, the apparatus may further comprise means for cooling at least a portion of the target area. The apparatus is preferably configured to automatically move the means for cooling across the target area such that cooling is applied essentially uniformly across the target area. To this end, the means for automatically moving the energy beam may be used as well or a separate unit for automatically moving the means for cooling may be provided.

In general, the means for cooling may be configured to reduce a temperature of at least a portion of the target area. For example, the means cooling may be configured to cool at least a part of the portion to which energy is delivered. In particular, the means for cooling may be configured to cool at least a part of the epidermal surface layer of a patient. In some embodiments, the means for cooling may comprise a chilled solid surface for contact cooling and/or a cryogenic spray cooling and/or forced air cooling.

Specifically, the apparatus, e.g., the means for automatically moving, may be further configured to move the means for cooling across the target area such that cooling is applied essentially uniformly across the target area. For example, the means for cooling may be moved essentially uniformly across the target area. In general, the means for delivering the energy beam may be moved together with the means for cooling. Alternatively, the means for cooling may be moved independently of the means for delivering the energy beam.

Automatically moving the means for cooling such that cooling is applied essentially uniformly across the target area ensures a coherent treatment of the target area. In particular, by automatically moving, the delivery of cooling does not rely on a possibly imprecise movement and/or coordination of arm and hand, as it is the case for handheld devices. This minimizes an overtreatment in the edges of the target area and thus reduces the risk of overexposure. Further, the automatically moving may comprise moving the means for delivering cooling according to (e.g., along) a predetermined path which is configured or determined for the target area. This guarantees that no part of the target area is omitted, minimizing the possibility of an undertreatment and thus optimizing effectiveness of the treatment.

Specifically, the means for automatically moving may be adapted to control, during moving the means for cooling, an effective distance of the means for cooling and/or an orientation of the means for cooling relative to the target area, preferably at least in part based on the at least one 3D image of the target area.

An effective distance of the means for cooling relative to the target area may comprise a spatial distance between at least a part of the means for cooling and a portion of the target area. For example, the portion of the target area may be the portion to which cooling is applied and/or to which energy is delivered. Similarly, the orientation of the means for cooling may comprise an orientation of at least a part of the means for cooling and a portion of the target area. Generally, the effective distance and/or the orientation of may be based on the at least 3D image of the target area. For example, effective distance and/or the orientation of the means for cooling may be controlled based on information about the target area associated with the at least one 3D image, e.g., a height and/or depth profile of the target area. In some embodiments, the means for automatically moving may be configured to maintain the means for delivering energy at a predefined effective distance and/or a predefined orientation. In particular, the means for automatically moving may be configured to maintain an essentially perpendicular orientation relative to the target area.

Controlling the effective distance and/or the orientation of the means for cooling relative to the target area may ensure that cooling is applied essentially uniformly across the target area. This minimizes an overtreatment in the edges of the target area and thus reduces the risk of overexposure. This guarantees that no part of the target area is omitted, minimizing the possibility of an undertreatment and thus optimizing effectiveness of the treatment.

Generally, the means for cooling may comprise a nozzle for cooling at least a portion of the target area with a fluid. Further, the means for cooling may be at least part of a cooling system. For example, the cooling system may be such as disclosed in EP 3 520 728 A1 and EP 4 098 217 A1 as well as EP 22 211909 (and their foreign family members), whose disclosure is incorporated herein in its entirety by reference. The cooling system may comprise at least one of a liquid reservoir, a pumping means, a gas reservoir, a gas compressor, gas valves, a gas pressure regulator, tubes for the gas, tubes for the liquid and a nozzle assembly. For example, the means for cooling may be the whole cooling system. Alternatively, the means for cooling may be a proper subset of the cooling system, preferably not including the liquid reservoir and/or the gas reservoir.

The means for cooling may for example comprise an outcoupler, e.g., in the form of an opening, a nozzle, etc. from which a fluid may be ejected, such as a liquid, a gas, a spray, etc. In these examples, the means for delivering energy may be coupled to a reservoir of the fluid, e.g., the liquid or the gas that may be stationary and/or external to the apparatus. The coupling may be implemented via one or more tubes, for example.

Using a nozzle, particularly a spray nozzle, for cooling at least a portion of the target area with a fluid has the advantage that the nozzle, i.e., the means for cooling, is not in direct contact with the target area, e.g., the skin of the patient. Furthermore, the cooling rate is significantly faster compared to cooling techniques such as cryogen spray cooling and cold air cooling. This enables a much faster movement of the means for moving and consequently of the means for delivering the energy beam across the target area. Therefore, the treatment time, i.e., the time needed to complete the treatment, can significantly be reduced, thus maximizing the comfort and well-being of the patient and thereby optimizing the treatment. Furthermore, reduced treatment times may enable the treatment of more patients in a given period of time, hence optimizing the capacity of the apparatus.

The means for automatically moving may be configured to move the energy beam based on a parameter of the energy beam.

In general, the means for automatically moving may move the energy beam based on a parameter of the energy beam. In particular, the means for automatically moving may move the means for delivering the energy beam based on a parameter of the energy beam. In other words, the movement of the energy beam across the target area may depend on at least one parameter of the energy beam. The parameter of the energy beam may comprise a width and/or diameter of the energy beam and/or an energy to be delivered per unit area and/or per unit time by the energy beam and/or an active time during which the means for delivering the energy beam deliver is to deliver the energy beam; and/or a focal length of the energy beam.

In addition, or alternatively, the means for automatically moving may be configured to move the energy beam based on a parameter of the cooling and/or a target treatment profile.

The means for automatically moving may move the energy beam based on a parameter of the cooling and/or a target treatment profile. In other words, there may be an interdependency between the movement of the energy beam across the target area and a parameter of the energy beam and/or a parameter of the cooling and/or the target treatment profile.

The parameter of the cooling may comprise a cooling strength to be delivered per unit area and/or per unit time by the means for cooling and/or an active time during which the means for cooling is to be active. For example, if the means for cooling comprise a nozzle for cooling at least a portion of the target area with a fluid, the parameter may comprise an amount of fluid to be delivered per unit area and/or per unit time by the nozzle.

The target treatment profile may be defined before the treatment is initiated and may specify the target treatment of the treatment area. The target treatment profile may comprise a target total energy to be received by the target area. In addition, or alternatively, the target treatment profile may comprise a target and/or maximal temperature of the target area and/or a duration of the treatment of the target area. In addition, or alternatively, the target treatment profile may further comprise exclusion and/or sensitive areas of the target area.

Specifically, the parameter of the cooling may be at least in part based on the parameter of the energy beam. In addition, or alternatively, the parameter of the cooling may be at least in part based on the target treatment profile.

In other words, there may be also an interdependency between the parameter of the cooling and the parameter of the energy beam and/or the target treatment profile.

By allowing for a dependence of the means for automatically moving on the parameter of the energy beam and/or the configuration of the means for cooling and/or the target treatment profile, the movement of the energy mean and/or the means for delivering the energy beam and/or the means for cooling can be adapted according to the respective parameters, which may lead to a reduced risk of overexposure or underexposure and to an improvement of the quality of treatment in general. Where the various means depend on each other (interact), a particularly effective and well-targeted treatment results.

In general, automatically moving the energy beam may comprise moving the energy beam across the target area in a continuous motion, preferably at a constant speed.

In general, the continuous motion may not comprise instantaneous changes in direction and/or orientation and/or speed and/or distance of the energy beam relative to the target area. In particular, the speed of the continuous motion may be non-zero during the treatment. For example, moving the energy beam across the target area in a continuous motion may comprise to move the means for delivering the energy beam in a continuous motion, preferably in a continuous motion across the target area.

In addition, or alternatively, automatically moving the means for cooling may comprise moving the means for cooling across the target area in a continuous motion, preferably at a constant speed.

For example, the speed of the energy beam and/or the means for cooling relative to the target area may be the rate of change in position with respect to all translational degrees of freedom or it may be the rate of change in position with respect to the planar components of movement only, e.g., the speed may not take changes in distance relative to the target area into account. In other words, the speed of the energy beam and/or the means for cooling may be the rate of change in position only with respect to the planar components of the movement.

The continuous motion of the energy beam may enable, support and/or enhance the essentially uniform delivery of energy across the target area. Similarly, the continuous motion of the means for cooling may enable, support and/or enhance the essentially uniform cooling across the target area. Therefore, the continuous motion of the energy beam and/or the means for cooling may contribute to minimizing the chances of burns and other adverse reactions, including skin irritation, redness, scars and swelling, thus improving the quality of the treatment.

In addition, or alternatively, automatically moving the means for delivering the energy beam may comprise moving the energy beam sequentially across the target area, wherein throughout a first phase of the sequence the energy beam is moved across a first portion of the target area at a first speed and throughout a second phase of the sequence the energy beam is moved across a second portion of the target area at a second speed. For example, the first speed may be different from the second speed.

In addition, or alternatively, automatically moving the means for cooling may comprise moving the means for cooling sequentially across the target area, wherein throughout a first phase of the sequence the means for cooling is moved across a first portion of the target area at a first speed and throughout a second phase of the sequence the means for cooling is moved across a second portion of the target area at a second speed. For example, the first speed may be different from the second speed.

In general, the first phase and/or the second phase may comprise a contiguous period. Similarly, the first portion and/or the second portion may be connected portions of the target area. In particular, the first portion may be a portion of the target area which is adj acent to the second portion. The energy beam and/or the means for delivering the energy beam may be moved throughout the first phase across the first portion at a first speed and throughout the second phase across the second portion of the target area at the second speed. Similarly, the means for cooling may be moved throughout the first phase across the first portion at a first speed and throughout the second phase across the second portion of the target area at the second speed.

Specifically, the first speed may be different from the second speed. In other words, the energy beam and/or the means for cooling is moved throughout the first phase across the first portion of the target area with a different speed than throughout the second phase across the second portion of the target area. For example, the speed in the first phase and/or the speed in the second phase may be constant, but mutually different. For example, the first speed may be zero, the second speed may be non-zero. In some embodiments, the first speed may vary in time according to a first function and/or the second speed may vary in time according to a second function. In this case a difference between the first speed and the second speed may imply that the first function and the second function are not identical.

Moving the energy beam and/or the means for cooling according to a first speed across a first portion may allow to apply two different treatments to the target area. For example, given that a fluence of the energy beam is the same for both portions, a first amount of energy is delivered to the first portion while a second amount of energy is delivered to the second portion. More precisely, the first speed in the first phase may be larger than the second speed in the second phase. Consequently, less energy may be delivered in the first phase to the first portion than in the second phase to the second portion. In other words, adjusting the speed may adjust the amount of energy delivered to at least a portion of the target area.

In general, the at least one parameter of the energy beam may comprise a first set of parameters throughout the first phase and a second set of parameters throughout the second phase. For example, the first set of parameters may be different from the second set of parameters. The first set of parameters and the second set of parameters may be such that energy is delivered to the target area by the energy beam in both phases. For example, the first set of parameters may prescribe the delivery of an amount of energy to at least a portion of the target area which is larger than the amount of energy which is delivered to the at least one portion of the target area according to the second set of parameters. The first set of parameters may differ from the second set of parameters in an energy to be delivered per unit area and/or per unit time and/or an active time during which energy is delivered. Alternatively, the energy beam and/or the means for delivering the energy beam may also be inactive (idle) with respect to the first set or the second set of parameters.

Similarly, the means for cooling may apply cooling according to a first set of parameters throughout a first phase and according to a second set of parameters throughout a second phase, wherein the first set of parameters may be different from the second set of parameters. The first set of parameters and the second set of parameters of the means for cooling may both be such that cooling is provided to the target area, i.e., the means for cooling may be active in both configurations. For example, the cooling of the target area according to the first set of parameters may provide more cooling of the target area as compared to the cooling of the target area according to the second set of parameters. In some embodiments where the means for cooling comprise a nozzle for cooling at least a portion of the target area with a fluid and/or an atomized liquid spray, the first set of parameters may specify that a first amount of fluid is to be ejected which is larger than a second amount of fluid that is specified to be ejected in the second set of parameters. Alternatively, the means for cooling may also be inactive (idle) with respect to the first or second set of parameters.

Adapting the energy beam, e.g., the parameters of the energy beam, such that the beam operates according to a first and a second set of parameters may allow to accommodate patient and/or target area specific conditions in the treatment. More precisely, a first portion of the target area may require a treatment according to a first set of parameters the energy beam while a second portion of the target area may require a treatment according to a second set of parameters of the energy beam. For example, the first portion may comprise skin and/or epidermis which is more sensitive to energy and/or heating while the second portion comprises skin and/or epidermis which is more robust. Consequently, more energy may be delivered to the first portion than to the second. Therefore, the treatment profile may be customized to the patient's needs individually and thus results in an optimized treatment. Similarly, adapting the means for cooling such that it can operate according to a first and a second set of parameters may allow to accommodate patient and/or target area specific conditions to the treatment.

Generally, the means for receiving the at least one 3D image may comprise a camera. For example, the camera may be a depth-sensing camera. In addition, or alternatively, the camera may be at least partially attached to the means for delivering the energy beam and/or the means for automatically moving.

For example, the depth-sensing camera may detect a depth and/or height based on Stereo Sensors, Time-of-Flight (TOF) calculations, Structured Light and/or LiDAR. The optical part of the camera, particularly the depth-sensing camera, may be less than $30 \times 30 \times 10$ mm. The camera may be at least partially attached to the means for delivering the energy beam and/or the means or automatically moving. For example, attaching the camera to the means for automatically moving may comprise that the camera is moved across the target area. Furthermore, attaching the camera to the means for delivering the energy beam may comprise that the camera acquires images of at least a part of the portion to which energy is delivered by the energy beam. In particular, by attaching the camera to the means for automatically moving and/or the means for delivering the energy beam, the movement of the means for delivering the energy beam and the movement of the camera across the target area may be correlated. In some embodiments, the camera may be a 3D Time-of-Flight (TOF) camera that may work by illuminating the at least a part of the target area with a modulated light source and observing the reflected light. The phase shift between the illumination and the reflection may be measured and translated to distance.

Depth-sensing cameras enable the apparatus to perceive the surfaces in three dimensions. In particular, a depth-sensing camera may allow for a real-time surface sensing of at least a part of the target area while the energy beam is moved across the target area. Thereby, moving the energy beam across the target area is based on a realistic and current information of at least a portion of the target area. Therefore, using a depth-sensing camera enhances the essentially uniformly delivery of energy across the target area by the energy beam and reduces the risk of over- and/or undertreatment of parts of the target area. Thus, using a depth-sensing camera optimizes the quality of the treatment. Furthermore, an important advantage of TOF camera is that the pulsed illumination light source is positioned in close vicinity to the corresponding detector, which allows for a reduces size of the entire sensor compared to stereo or triangulation based 3d sensors. This enables a compact integration of the TOF camera to the apparatus and/or the means for automatically moving and/or the means for delivering the energy beam. In particular, attaching the camera, particularly the TOP camera, to the means for automatically moving and/or the means for delivering the energy beam assures that the camera measuring range is unobstructed, which this is not true if the camera is positioned separately at fixed position relative to the patient.

Specifically, the camera may be attached such that its optical axis essentially forms a right angle with respect to the energy beam. Additionally, the apparatus may further comprise a dichromatic mirror, wherein the dichromatic mirror is arranged such as to transmit the energy beam and to at least partially reflect radiation to which the camera is sensitive to.

The optical axis of the camera may comprise an axis of the camera through which radiation, e.g., light, enters the camera and/or impinges the camera. For example, the optical axis may be an axis that passes through a geometrical centre of an optical system of the camera. In particular, the optical system may comprise a camera lens, a microscope and/or a telescopic sight. In general, the optical axis may define the path along which light propagates through the camera, up to first approximation.

The apparatus may further comprise a dichromatic mirror. A dichromatic mirror may be an optical element which reflects a first light and transmits a second light, preferably with almost no absorption. For example, the dichromatic mirror may be arranged such as to transmit the energy beam and to at least partially reflect radiation to which the camera is sensitive to. In particular, the dichromatic mirror may be configured and/or arranged such that the energy beam may be transmitted by the dichromatic mirror such that energy is delivered to the at least portion of the target area. In particular, the dichromatic mirror may be such that the energy beam after passing through the dichromatic mirror is essentially the same as before passing through the dichromatic mirror. That is, the energy distribution and/or the intensity and/or the polarization and/or the geometry of the energy beam after passing through the mirror may be essentially the same as before passing through the mirror. Furthermore, the dichromatic mirror may at least partially reflect radiation to which the camera is sensitive to. In other words, radiation and/or light may enter the camera which has been reflected by the mirror.

By using a dichromatic mirror and by attaching the camera such that its optical axis essentially forms a right angle with respect to the energy beam, the energy beam can be delivered to the at least portion of the target area while, at the same time, the camera can acquire the at least one 3D image of the target area, although the optical axis forms a right angle with respect to the energy beam. Due to the perpendicular orientation of the optical axis of the camera, a measuring range of the camera can be enlarged. The enlarged measuring range contributes to an optimized 3D image of the target area and thus to an improved treatment, e.g., an improved essentially uniformly delivery of energy across the target area.

In addition, or alternatively, the camera may be attached to the means for delivering the energy beam and/or the means for automatically moving such that an angle between the energy beam and an optical axis of the camera is at least 1°, preferably at least 10°, more preferably at least 20°, most preferably at least 30° and/or such that an angle between the energy beam and an optical axis of the camera is at most 40°, preferably at most 35°, most preferably at most 30°. In addition, or alternatively, the camera may be attached to the means for delivering the energy beam and/or the means for automatically moving such that a horizontal and/or vertical distance of the camera and the energy beam and/or the means for delivering the energy beam is at least 1 mm, preferably at least 5 mm, most preferably at least 10 mm and/or at most 60 mm, preferably at most 55 mm, most preferably at most 50 mm.

In general, the apparatus may be configured to adaptively control, while moving the energy beam, a position relative to at least a portion of the target area to which the energy beam is delivered, the effective distance and/or the orientation of the energy beam relative to at least a portion of the target area, wherein the adaptive control is at least in part based on an image of at least a portion of the target acquired while moving the energy beam.

In general, a position of the energy beam and/or the means for delivering the energy beam and/or the means for automatically moving relative to the target area may comprise a position with respect to the surface defined by the target area. For example, the position may comprise a two-dimensional position, particularly a two-dimensional position with respect to a plane associated with the target area. In some embodiments, moving the energy beam across the target area may be based on the position and the effective distance. Generally, the position may be defined with respect to a plane which is essentially perpendicular to a direction with respect to which the effective distance is measured.

The adaptive control of the position to at least a portion of the target area to which the energy beam is delivered, the adaptive control of the effective distance and/or the adaptive control of the orientation of the energy beam relative to the at least portion of the target area may be at least in part based on an image of at least a portion of the target area acquired while moving the energy beam. In other words, the apparatus may acquire while moving the energy beam across the target area an image of at least a portion of the target area and control the position, the effective distance and/or the orientation based on the image. For example, the image may comprise at least a part of the at least portion of the target area to which energy is delivered by the energy beam, e.g., to which energy is delivered while acquiring the image. In general, the adaptive control may comprise a repositioning and/or a reorientation and/or a change of the effective distance relative to the target area.

In other words, the image of the at least portion of the target area acquired while moving the energy beam, may provide feedback in the real-time control loop during treatment, e.g., during moving the energy beam across the target area, to compensate for patient's movement, e.g., breathing. In particular, the adaptive control may ensure that the actual movement of the patient is taken into account and that the orientation of the energy beam relative of the target area does not deviate from the predefine, e.g., perpendicular orientation, by more than a threshold. By taking into account the actual movement of the patient a essentially uniformly delivery of energy across the target area can be ensured. In particular, movements of the patient that may change a position of the target area relative to the apparatus and/or an effective distance and/or orientation of the energy beam relative to the target area can be detected and compensated. Thereby, the risk of over- and/or undertreatment of parts of the target area is minimized and the quality of the treatment maximized.

Specifically, the adaptive control may be at least in part based on a height of the at least a portion of the target area to which the energy beam is delivered. For example, the height may be based on an image from a height sensor of the apparatus and/or from the means for receiving the 3D image.

The adaptive control may be at least in part based on the height of the at least portion of the target area to which the energy beam is delivered. For example, the hight of the portion may have changed during the treatment of the target area. In general, the height of the at least one portion may comprise a height and/or depth profile of the surface of the at least one portion. In particular, the height of the at least portion may allow to compute an inclination and/or a gradient with respect to the at least portion. In some embodiments, the orientation of the energy beam may be based on the height of the at least portion. For example, based on the height, the means for automatically moving moves at least a portion of the means for delivering the energy beam such as to maintain a predefined orientation of the energy beam relative to the at least portion of the target area.

In general, the height may be based on an image from a height sensor of the apparatus and/or from the means for receiving the 3D image. For example, the means for automatically moving and/or the means for delivering the energy beam may comprise the height sensor. In addition, or alternatively, the height may be based on an image from the means for receiving the 3D image. For example, when the means for receiving the 3D image comprise a camera, particularly a depth-sensing camera, the height may be based on at least one image acquired by the camera. In particular, the camera may be arranged in a vicinity of the means for delivering the energy beam.

By using a height sensor of the apparatus and/or the means for receiving the 3D image for receiving the height of the at least portion of the target area to which energy is delivered, a current height of the at least portion can be obtained.

In addition, or alternatively, the adaptive control may be at least in part based on a temperature profile of the at least a portion of the target area to which the energy beam is delivered. For example, the temperature profile may comprise a temperature and/or thermal field.

Generally, a temperature profile of the at least portion of the target area may comprise at least two temperature values associated with two different points of the target area. The adaptive control may be at least partially based on a temperature profile of the at least portion of the target area to which the energy beam is delivered. For example, the energy profile may comprise a temperature and/or thermal field. In particular, the temperature profile may comprise a temperature distribution of at least a part of the target area. For example, the temperature profile may comprise a temperature distribution of the at least portion of the target area to which energy is delivered by the energy beam. Furthermore, the temperature profile may comprise a temperature distribution of a part of the target area to which energy beam will be moved. For example, the temperature profile may comprise a temperature distribution of a part of the target area to which energy beam will be moved within 1 minute, preferably within 30 s, more preferably within 20 s, most preferably within 10 s. In addition, or alternatively, the size of the part of the target area to which energy beam will be moved may be based on the diameter of the energy beam. In some embodiments, the temperature profile may comprise monitoring a temporal evolution of the temperature distribution of at least a part of the target area. In particular, monitoring the temporal evolution of the temperature distribution may comprise determining a heating and/or cooling rate of at least a portion of the target area.

Specifically, the control of the position relative to at least the portion of the target area to which the energy beam is delivered may be based on the temperature profile. For example, based on the temperature profile in-plane movements of the at least portion of the target area and/or the target area may be detected. For example, based on the temperature profile, e.g., the temperature distribution of the at least portion, recognizing representative features of the (spatially) distribution may be detected. For example, recognizing representative features may comprise a temperature peak, a temperature minimum, a temperature pattern and/or a shape of temperature peaks and/or temperature minimums. Generally, based on the recognizing representative features the position relative to at least the portion of the target area to which the energy beam is delivered may be controlled. In particular, an in-plane movement of the recognizing representative features may be tracked over time. The tracking may be based on various known algorithms, e.g., digital image correlation, optical flow and/or feature detection.

Using the temperature profile for the adaptive control, particularly for an adaptive control of the position, has the advantage that no additional markings, e.g., such as stickers or drawings, are required on the skin. Consequently, the process is cleaner and undisturbed. In particular, if a colour camera is used, the markers must be positioned outside the target area. For this reason, the viewing area of the colour camera should be larger than the target area so that the camera cannot be positioned at the means for delivering the energy beam. The colour camera must therefore be positioned in a separate (distant) location, which complicates the system considerably.

Generally, the apparatus may further comprise means for determining a temperature of at least a portion of the target area. For example, the means for determining a temperature may comprise an infrared camera.

The means for determining the temperature of at least a portion of the target area may determine the temperature of the at least portion to which energy is delivered. For example, the temperature profile may be based on data and/or information acquired by the means for determining the temperature. In general, the means for determining the temperature may be attached at least partially to the means for delivering the energy beam and/or the means for automatically moving. For example, the means for determining the temperature may be comprised in the means for delivering the energy beam. In particular, the means for determining the temperature may be moved across the target area during the treatment.

Generally, the parameter of the energy beam and/or the means for delivering the energy beam and/or the means for cooling may depend on data and/or information obtained by the means for determining the temperature.

In some embodiments, the means for automatically moving may comprise at least one robotic arm. For example, the robotic arm may be movable with respect to at least 3 degrees of freedom.

For example, the at least one robotic arm may at least partially comprise the means for delivering the energy beam and/or the means for cooling. In particular, one robotic arm may at least partially comprise both, the means for delivering the energy beam and the means for cooling. In addition, or alternatively, there may be a first robotic arm at least partially comprising the means for delivering the energy beam and a second robotic arm at least partially comprising the means for cooling. In some embodiments, the means for delivering the energy beam and/or the means for cooling may be attached to the at least one robotic arm. For example, the means for delivering the energy beam and/or the means for cooling may be at least partially attached to a front end of the at least one robotic arm. Furthermore, the robotic arm may be movable with respect to at least 3 degrees of freedom. Preferably, the robotic arm may be movable with respect to at least 4 degrees of freedom, more preferably at least 5 degrees of freedom, most preferably at least 6 degrees of freedom.

Using a robotic arm for automatically moving may allow a high degree of flexibility in moving the means for delivering the energy beam and/or the means for cooling. In particular, a robotic arm may allow to quickly, reliably and easily adapt to arbitrary target areas.

Moreover, the means for automatically moving may generally be any apparatus that allows to move an object along a predetermined path. A robotic arm, as well known in the art, is just one example for such a means for automatically moving. Other apparatuses may be used as well. For example, an apparatus comprising a terminal end that is movable along a strut, which is in turn movable relative to one or more further struts, may be used similar to a printer head or a crane hook.

In general, the energy beam may comprise a laser beam. For example, the laser beam may be generated by a Nd:YAG laser. In addition, or alternatively, the laser beam may be generated by an Alexandrite laser. In addition, or alternatively, the laser beam may be generated by an Er:YAG laser. In addition, or alternatively, the laser beam may be generated by a $CO_2$ laser.

Using a laser beam as the energy beam allows for a precise treatment of the target area as the laser beam has an energy distribution that is spatially very confined. In particular, laser light can be accurately focused to small spots with very high energy. Further, a laser allows for precise adjustment of result-effective variables such as fluence and/or wavelength. In addition, lasers are relatively cost-efficient.

Generally, the apparatus may comprise a footswitch wherein the footswitch is configured to at least partially control the means for delivering the energy beam and/or the means for cooling and/or the means for automatically moving. In addition, or alternatively, the apparatus may comprise a handheld control-device wherein the handheld control-device is configured to at least partially control the means for delivering the energy beam and/or the means for cooling and/or the means for automatically moving.

For example, the footswitch may be configured to start and/or to stop the treatment of the target area. For example, the footswitch may start and/or stop the delivery of energy by the energy beam to the target area and/or the cooling by the means for cooling and/or the moving by the means for automatically moving. Generally, the footswitch may be connected to the means for delivering the energy beam and/or the means for cooling and/or the means for automatically moving. The connection may comprise a wired and/or wireless connection. If the energy beam is a laser beam and the laser beam is generated by a laser system, the footswitch may be connected to the laser system. In some embodiments, there may be a first footswitch configured to at least partially control the means for delivering the energy beam and/or the energy beam and/or the means for cooling and a second footswitch for at least partially control the means for automatically moving. In addition, or alternatively, a single footswitch may be used to control both, the means for automatically moving and the means for delivering the energy beam and/or the energy beam and/or the means for cooling.

Generally, the foot switch and/or the handheld controlled switch makes the treatment more adaptable to the needs of both, the therapist and/or the patient. For example, the therapist may stop and/or start the treatment of the target area according to demand and may thus contribute to the essentially uniformly delivery of energy to the target area. In addition, the footswitch contributed to the safety of treatment as the therapist may intervene the treatment if necessary.

In some embodiments, the apparatus may further comprise at least one user interface for controlling the means for delivering the energy beam and/or the means for cooling and/or the means for automatically moving. For example, the at least one user interface may be configured to receive a user input identifying the target area within a graphic representation of at least a portion of the patient. In particular, the graphical representation may be based on the at least one 3D image of the target area.

The apparatus for treatment of a target area may further comprise at least one user interface for controlling the means for delivering the energy beam, the means for cooling and/or the means for automatically moving. Therein, the at least one user interface may be configured to receive a user input identifying the target area. Specifically, the user input may identify the target area within a graphic representation of at least a portion of a patient. The graphical representation may be based on the at least one 3D image of the target area. For example, the user input may comprise markings of the corners of the target area on the user interface based on the at least one 3D image. In particular, the user may specify on a touch screen of the user interface the corners and/or a boundary of the target area. For example, the target area may comprise a convex hull of the corners and/or the boundary.

The user input may identify the target area among a set of possible target areas, which may be listed and/or graphically represented by the user interface. For example, the user interface may be used to configure the means for automatically moving and/or specify the parameters of the energy beam and/or the parameters of the means for cooling. The means for automatically moving may be configured based on the user input identifying the target area.

Similarly, the parameters of the energy beam and/or the parameters of the means for cooling may be specified based on the user input identifying the target area. In some embodiments the graphical representation may be generated based on at least one image captured with at least one camera. More precisely, the user interface may comprise an image recognition software. In this case, the listed and/or graphically represented possible target areas within the user interface may correspond to abstract and/or virtual target areas which are identified by virtue of the image recognition software and the at least one image with the corresponding target area of the patient.

By identifying the target area within a graphical representation of at least a portion of the patient, the identification may be unambiguous and easy to carry out by a user. This is especially the case when the graphical representation is generated based on the at least one 3D image of the target area. In particular, this may allow for a precise and individual configuration of the treatment area. Configuring the means for automatically moving and/or the means for delivering the energy beam and/or the parameters of the energy beam and or the parameters of the means for cooling based on the user input may allow to generate the configuration for the target area individually.

A second aspect relates to a control method. The method comprises receiving at least one 3D image of a target area of a patient for treatment by an energy beam. The method further comprises determining a configuration according to which to move the energy beam relative to the target area based on the at least one 3D image. The configuration may comprise an effective distance of means for delivering the energy beam and/or an orientation of the energy beam relative to the target area. The method further optionally comprises moving the energy beam across the target area based on the determined configuration, such that energy is delivered essentially uniformly across the target area.

In some examples, the control method is part of a method for treatment of the target area by the energy beam, for cosmetic or therapeutical purposes. In some examples, moving of the energy beam is carried out by moving the means for delivering the energy beam.

A third aspect is directed to a computer program comprising instructions for performing the above-described method when the instructions are executed.

Generally, the present disclosure also comprises that any aspect described herein, regardless of whether described with reference to an apparatus, a method or a computer program, may be implemented as a function of an apparatus, a step of a method or an instruction of a computer program.

Whether aspects are implemented as hardware or software means depends upon the particular application and design constraints imposed on the overall system. By way of example, an element, or any portion of an element, or any combination of elements may be implemented as a processing system that may include one or more processors. Examples of processors include microprocessors, microcontrollers, graphics processing units (GPUs), central processing units (CPUs), application processors, digital signal processors (DSPs), reduced instruction set computing (RISC) processors, systems on a chip (SoC), baseband processors, field programmable gate arrays (FPGAs), programmable logic devices (PLDs), state machines, gated logic, discrete hardware circuits, and other suitable hardware configured to perform the various functionality described throughout this disclosure. One or more processors in the processing system may execute software. Software shall be construed broadly to mean instructions, instruction sets, code, code segments, program code, programs, subprograms, software components, applications, software applications, software packages, routines, subroutines, objects, executables, threads of execution, procedures, functions, etc., whether referred to as software, firmware, middleware, microcode, hardware description language, or otherwise.

Accordingly, in one or more exemplary embodiments, the functions described may be implemented in hardware, software, or any combination thereof. If implemented in software, the functions may be stored on or encoded as one or more instructions or code on a computer-readable medium. Computer-readable media includes computer storage media. Storage media may be any available media that can be accessed by a computer. By way of example, and not limitation, such computer-readable media can comprise a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that can be used to store computer executable code in the form of instructions or data structures that can be accessed by a computer.

It is noted that any combination of features that have been described above as belonging to certain embodiments/aspects of the present invention is also an embodiment of the present invention, provided such a feature combination is feasible, i.e., does not lead to any contradictions.

Short description of the figures

In the following, exemplary embodiments of the invention are described with reference to the figures. The figures show:

Fig. 1A: Exemplary embodiment of an apparatus for treatment of a target area of a patient showing a laser system and a part of a robotic arm;

Fig. 1B: Exemplary embodiment of an apparatus for treatment of a target area of a patient showing a robotic system comprising a robotic arm;

Fig. 2A: Exemplary embodiment of a first positioning configuration of the means for receiving the at least one 3D image and the means for delivering the energy beam;

Fig. 2B: Exemplary embodiment of a second positioning configuration of the means for receiving the at least 3D image and the means for delivering the energy beam;

Fig. 2C: Exemplary embodiment of an optical filter arrangement for the 3D camera;

Fig. 3: Exemplary illustration of a calibration of the apparatus comprising a reference geometry;

Fig. 4: Exemplary embodiment of a pre-scanning procedure of the apparatus;

Fig. 5: Exemplary embodiment of a contour registration procedure;

Fig. 6A: Exemplary illustration of the calculated path for the robotic arm before the treatment;

Fig. 6B: Exemplary illustration of the calculated path for the robotic arm during the treatment;

Fig. 7: Illustration of the different covering modes according to the present invention;

Fig. 8A: Comparison of the cooling rates during CAC and DMC cooling;

Fig. 8B: Exemplary illustration of the principle of the inverse cooling mechanism;

Fig. 9:        Exemplary illustration of (thermal) features based on thermal imaging for in-plane movement detection.

Detailed description of preferred embodiments

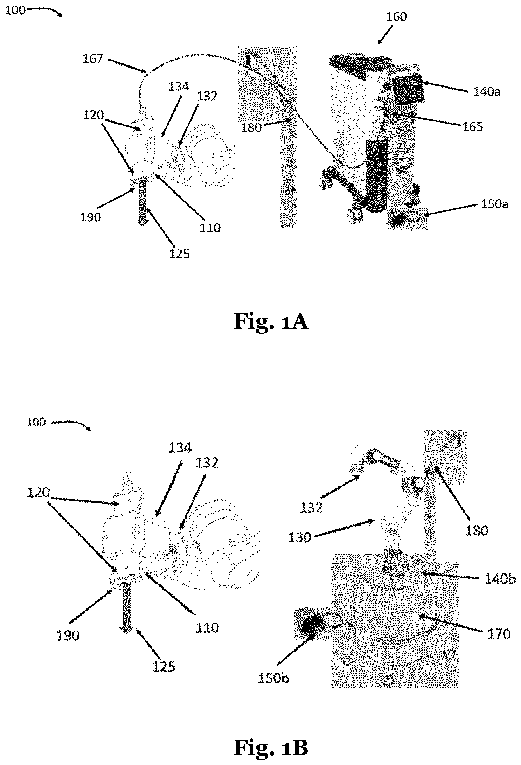

Figure 1A and 1B show an exemplary embodiment of an apparatus 100 for treatment of a target area of a patient by an energy beam. The apparatus comprises a 3D time-of-flight camera 110, a laser handpiece 120 for delivering a laser beam 125 to at least a portion of the target area. The apparatus further comprises a robotic arm 130, user interfaces 140a, 140b, footswitches 150a, 150b, a laser system 160, a robot system 170 with a housing, a laser fibre antenna holder 180 and a temperature sensor 190.

The robot system 170 comprises a robotic arm 130 which comprises a robot system head 134 and a robot system end 132.

Further, the laser system 160 comprises a laser source 165. The laser optical fibre holder 180 is configured to hold a laser optical fibre 167, which at least partially connects the laser handpiece 120 (mounted on the robotic system's head 134) with the laser source 165. In the illustrated embodiment, the footswitches 150a and 150b coincide, i.e., a single footswitch is used for controlling both, the laser system 160 and the robotic system 170. Alternatively, the footswitches 150a, 150b may be separate footswitches.

It is to be understood here that for simplicity the general term laser handpiece is used since in certain embodiments a standard physician's laser handpiece is mounted (detachably) on the robot's head. However, the term laser handpiece may denote also a special optical and mechanical assembly specifically designed to be used mainly when mounted on the robotic system head 134. Instead of a handpiece also other units may be used for the laser, e.g., units that are permanently attached to the robot's head.

Generally, the laser system 160 may comprises the laser system's main housing comprising the laser source 165 and laser power and control electronics, laser system's processor and the user interface 140a as well as the laser system's footswitch 150a. Furthermore, the laser system may comprise the laser optical fibre 167 and the laser handpiece 120. For example, the laser handpiece 120 may comprise a Fotona laser handpiece R35 that may be connected via the laser optical fibre 167 to a Fotona laser device AvalancheLase® operating at the Nd:YAG (1064 nm) laser or Alexandrite laser (755 nm) wavelength.

The robot system 170 comprises the robotic arm 130, robot's main housing, the robot system's processor and a user interface 140b. Furthermore, the robotic arm 130 may be a 6-7 DOF (Degrees of Freedom) robotic arm mounted on the robot system's housing. The robot system head 134 may be mounted on the robot system end 132. In general, the robotic system may further comprise the robot system's footswitch 150b. In general, the robotic system head 134 may thus represent the robotic system end effector, a term which may be used in robotics to represent a peripheral device that attaches to the robot's end, allowing the robot to interact with its task. For example, the robotic system may comprise a Franka Emika Panda robot.

The robotic arm 130 is configured to move the laser beam 125 across the target area such that energy is delivered uniformly across the target area. In particular, the apparatus is adapted to control, during moving of the laser beam 125, an effective distance of the laser handpiece 120 and/or an orientation of the laser beam 125 relative to the target area based on the at least one 3D image of the target area.

Generally, the control of the effective distance may comprise a control of a spatial distance to the target area and/or a control of a focus of the laser beam 125. In addition, or alternatively, the control of the orientation may comprise a control of an orientation of the laser handpiece 120 relative to the at least portion of the target area.

The 3D time-of-flight camera 110 may be configured to acquire at least one 3D image of at least a portion of the target area. For example, the 3D time-of-flight camera 110 may comprise a Pico Flexx time-of-flight (TOF) camera. For example, the 3D time-of-flight camera may capture point clouds at 25 Hz.

Specifically, the robotic system 170 and the 3D camera 110 may be connected to a processing unit customized to implement the control schemes described herein, and to send commands at a frequency of 1kHz to a cartesian velocity controller of the robot system. In some examples, the control unit may be integrated into the robotic system 170. In other examples, the control unit may comprise an external unit, e.g., an industrial computer, such as an Axiomtec, MANO561, intel Core i5, 16 GB RAM, connected to the robotic system 170 through a Robot Operating System (ROS) Noetic.

The laser beam 125 may have a finite width and impinges on the surface of tissue. On the tissue surface, the laser beam 125 emitted from the laser handpiece 120 thus gives rise to a spot S wherein the spot S can be defined as the region of the tissue surface within which 90 % of the total energy of the laser pulse or of the CW laser power is delivered to the tissue (i.e., the spot S corresponds to the smallest region on the tissue surface so that, during the delivery of the laser pulse, 90% of the total energy of the laser pulse or of the laser power is delivered to this region). Defining the direction of the sweeping of the laser beam 125 across the surface as the direction x, then the width of the spot in the x direction (i.e., $D_x$) may in general be different from the width of the spot $D_y$ in the y direction. For circular or square shaped spots, however, $D_x = D_y = D$. Spot size $D_x$ larger than 2 mm and smaller than 40 mm, calculated from the maximal robot speed. In some embodiments, the laser spot size may be in the range between 5 mm and 15 mm.

Generally, the laser energy may be delivered to the laser handpiece 120 via an optical delivery system (e.g., the laser optical fibre 167) from a laser source 165 contained in a separate laser system 160. In other embodiments, the laser system may be integrated into the robot system's main housing. In further embodiments, the laser source 165 and/or at least parts of the laser power and control electronics may be integrated into the robotic system head 134. In some of the embodiments, the laser handpiece 120 may be a manual handpiece with a fixed output beam direction, or a scanning handpiece capable of scanning the output beam over a certain pattern of beam angles. For example, the laser handpiece 120 may be configured to at least partially change the direction of the laser beam by optical elements. In particular, the laser handpiece may at least partially change the direction of the laser beam 125 by moving and/or rotating a mirror. In addition, or alternatively the laser handpiece 125 may comprise an acoustooptic modulator adapted to change the direction of the laser beam 125, preferably by an oscillating electric signal.

It is to be understood that the laser spot and/or laser beam 125 may not exhibit a homogeneous energy or power distribution across the spot area. For example, in certain embodiments the energy may be delivered to the tissue in a "patterned" or fractional shape, wherein the laser beam irradiates a number (M) of individual circular spots S' within the treatment area S whereas each spot S' having the size (e.g., diameter) D' is separated from a neighbouring spot by a certain distance. Patterned handpieces, such as the Fotona PSO3 handpiece have the spot size D' in the range of $0.3\,mm \leq D' \leq 1.5\,mm$, while the fractional handpieces such as Fotona FS01 handpiece have the spot size $D' \leq 0.3\,mm$. Further embodiments of patterned beam profiles, including a honeycomb pattern are described in the European patent application "Apparatus and method for patterned laser treatment of a tissue surface" with application number 23174965.6 filed on May 23, 2023.

Since surface procedures involve a real-time surface sensing problem of the human body, and an adaptive control problem (adapting to small movements such as breathing), a depth-sensing camera 110 (i.e., a depth camera) may represent a suitable choice for the 3D shape acquisition device. Depth-sensing camera 110 enables the robot to perceive the surfaces in three dimensions. Depth cameras typically detect depth using Stereo Sensors, Time-of-Flight (TOF) calculations, Structured Light, or LiDAR. In one of the preferred embodiments the image acquisition of choice is a 3D time-of-flight (TOF) camera that works by illuminating the scene with a modulated light source and observing the reflected light. The phase shift between the illumination and the reflection is measured and translated to distance.

The important advantage of the TOF camera 110 is that the pulsed illumination light source is positioned in close vicinity to the detector (such as RF-modulated light sources with phase detectors, Range gated imagers, or Direct Time-of-Flight imagers), thus the sizes of entire sensor are much smaller comparing to stereo or triangulation based 3d sensors. The optical part of TOF camera 110 may be less than $30 \times 30 \times 10\,mm$ (see for example flexx2 camera from PDMTEC, https://3d.pmdtec.com/en/3d-cameras/flexx2/). While typical size of stereo or triangulation camera system is >100 mm in longest dimension.

This enables a compact integration of the TOF camera 110 to the laser handpiece 120, which means it may be moved together with the laser handpiece 120. Further, this configuration assures that the 3D camera measuring range is unobstructed. However, this is not true if the camera is positioned separately at fixed position relative to the patient.

Generally, the apparatus 100 may further comprise means for cooling at least a portion of the target area. In particular, the robotic arm 130 may be configured to move the means for cooling across the target area such that cooling is applied essentially uniformly across the target area. For example, the laser handpiece 120, e.g., the Fotona R35 handpiece, may be equipped with a cooling outlet, e.g., a dry-molecular-cooling water spray-based skin cooling outlet. In addition, or alternatively, the laser handpiece 120 may further comprise a temperature sensor, e.g., a Fotona Matrix-View® temperature sensor.

In some examples, the laser device may deliver a pointing beam through the same optical fiber.

In general, the treatment of the target area of the patient may be divided into i) spatially "localized" surface procedures such as the treatment of localized vascular and pigmented lesions or scars where the laser radiation is within the treated surface area preferably delivered precisely to where the target is located (e.g., a vessel), and ii) "covering" surface procedures where generally the laser radiation is delivered in an approximately homogeneous manner over a larger tissue area.

In some embodiments, covering surface procedures (for short referred to also as the "covering procedures") may comprise aesthetic procedures such as hair removal (for example, with Nd:YAG (1064 nm), Alexandrite (755 nm), or diode (808 -1100 nm) lasers), or IPL Intense Pulse Light (pulse light (400-1200 nm), skin toning, skin whitening and removing pigmented lesions, including tattoo treatments (for example, with Q-switched or pico Nd:YAG (1064 nm and 532 nm) or Alexandrite (755 nm) lasers), vascular treatments (for example, with long pulse Nd:YAG (1064 nm and 532 nm) laser), skin resurfacing, scar reduction, skin tightening, skin rejuvenation, intense heat shock biomodulation (i-HBM) (for example with Nd:YAG (1320 nm), Nd:YAP (1340 nm), diode laser (1400-2000 nm), Er:glass (1540 nm), Ho:YAG (2100-2200 nm), erbium fiber (2910 nm), Er,Cr:YSGG (2790 nm) or Er:YAG (2940 nm) laser), transdermal lipolysis (for example with Nd:YAG (1064) or diode (1060 nm) laser), low level laser therapy (LLLT) or piano level laser therapy (PLLT and HILT) (for example with Nd:YAG or diode lasers), or hair growth stimulation (for example with Er:YAG (2940 nm) laser). Examples of mucosa covering procedures include vulva whitening and snoring treatments (for example with Er:YAG (2940 nm) laser).

Examples of exposed tissue surface procedures include tissue debridement and ablation (for example with Er:YAG (2940 nm) laser), coagulation (for example with $CO_2$ (9000-11000 nm) laser), and disinfection (for example with blue or blue-violet (380-500 nm) diode laser). In general, by selecting appropriate output parameters of the light source device, such as spot size, pulse duration, pulse power or average power, any of the above procedures can be performed with a light and/or laser device with any wavelength in the range of 200 nm-11000 nm.

Generally, the covering surface procedures described herein may also be performed with non-optical EBDs (Energy Based Devices), such as ultrasound, radiofrequency, or plasma-based devices.

In some embodiments, covering procedures may comprise a stamping and/or a brushing ("in-motion") technique. With the "stamping" technique the laser handpiece may be positioned over the treated skin from spot to spot with or without any substantial overlapping, and a relatively high laser energy is delivered to each of the spots while moving from spot to spot with a relatively low repetition rate (typically lower than 2 Hz). For example, this technique may most commonly being used for hair removal with pulsed Nd:YAG (1064 nm) and Alexandrite (755 nm) lasers since these lasers are able to deliver high energy laser pulses with sufficiently short pulse durations (~0.3-25 ms). Using this technique, hair follicles are due to their higher absorption of laser light in comparison to the surrounding skin matrix, selectively over-heated with single individual pulses delivered to each individual laser spot.

On the other hand, the brushing technique may involve lower energy laser pulses delivered at higher pulse rates (typically 3-10 Hz) with the laser beam in a constant movement forward and backward (for example, at a speed of 2-3 cm/s), typically with some level of overlap, until a sufficiently high cumulative energy is delivered to the whole treated area. In hair removal, continuous brushing technique may be more often used with diode lasers since they are typically not capable of delivering high energy single pulses with short enough pulse durations, however due to reduced pain this technique has been used also with pulsed Nd:YAG (1064 nm) and Alexandrite (755 nm) lasers. The destruction of hair follicles may result from the gradual temperature increase of the skin matrix and hair follicles during repeated laser irradiations, relatively indiscriminately delivered over the whole treatment area.

Alternatively, the brushing technique may be used with continuous wave or quasi continuous wave (QCW) lasers, e.g., CW diode lasers or QCW Nd:YAG laser, the final goal of the procedure being that the treated area is "covered" approximately homogeneously with a desired cumulatively delivered energy.

In general, the laser system and the robotic system may interact according to different configurations. A configuration may comprise how the robot system and the laser system are combined to constitute a joint robotic laser system.

In some embodiments, the robotic laser system may be a dual independent robotic laser system. A dual robotic laser system may comprise that the laser system and the robotic system are i) dual e.g., with the laser system and the robot system in physically separate housings, with separate processors and user interfaces. A dual robotic laser system may further comprise that the laser system and the robot system are independent e.g., where the two systems do not communicate with each other, and thus operate in most instances independently of each other. The only common feature is that they are both controlled by the same on/off footswitch and/or a handheld control device.

In addition, or alternatively, the robotic laser system may be a dual interacting laser system. The robotic laser system is i) dual, with the laser system and the robot system in physically separate housings, and may be ii) interacting, where the two systems are communicating with each other, for example by sharing their status and settings.

In addition, or alternatively, the robotic laser system may be a single independent robotic laser system. Being a single independent robotic laser system may comprise a robotic laser system that is i) single, e.g., with the laser system and the robot system in the same housing, however with separate processors and user interfaces, and ii) independent, e.g., where the two systems do not communicate with each other, and thus operate in most instances independently of each other. The only common feature is that they are both controlled by the same on/off footswitch and/or a handheld control device.

In addition, or alternatively, the robotic laser system may be a single interactive robotic laser system. Being a single interactive robotic laser system may comprise that the robotic laser system is i) single with the laser system and the robot system in the same housing, with separate or joint processors and separate or joint user interface, and ii) interacting, where the two systems are communicating with each other, for example by sharing their status and settings.

Generally, an advantage of the dual system may be that the laser handpiece and the robot can be more optimally positioned in relation to the patient. For example, the laser handpiece can be positioned on one (e.g., left) side of a bed, and the robot can be positioned on the other (e.g., right side of the bed). Alternatively, the robot can be positioned at the front of the bed (at the patient's head- for example for facial or head treatments) or at the end of the bed (at the patient's feet- for example, for hair removal on legs). Both devices can be moveable, e.g., on wheels.

An advantage of the dual independent system is that practically any laser device can be connected to the robot which can function as a universal robotic solution.

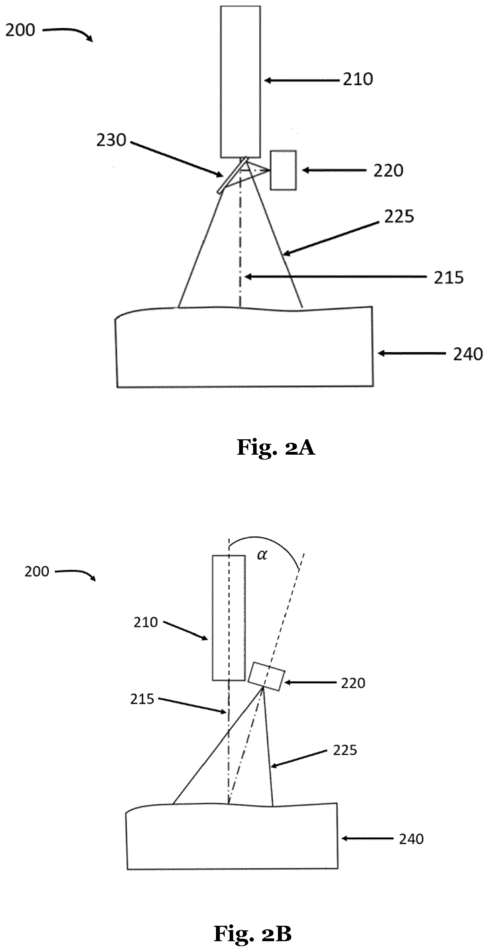

Figure 2A and 2B illustrate different configurations how the 3D camera 220 can be arranged with respect to the laser handpiece 210. As illustrated in Figure 2A, the 3D camera 220 is coupled coaxially with laser beam 215 through dichromatic mirror 230 which transmits the laser beam 215 towards at least a portion of the target area 240 and reflects the light 225 used by depth camera 220.

Alternatively, as illustrates in detail in figure 2B, the 3D camera 110 may be positioned at 10 to 50 mm horizontally and vertically relative to the beam exit of the laser handpiece 120, approximately parallel, with angle $\alpha$ preferably in a range from $\alpha = 10°$ to 30°. However, the accuracy of 3D measurements by any optical means may be degraded when the angle of measuring direction relative to the surface normal is increasing. The error is increasing approximately via

$$\text{DepthError} = \frac{C}{\cos \alpha}$$

where a is the angle between measuring direction and the surface normal, and C is measuring error in case of perpendicular measuring direction. So, it is obvious that $\alpha$ must be as small as possible to prevent excessive measurement errors. For example, if $\alpha = 10°$ and C=1 mm, the DepthError is increased by 1.5%, while in case of $\alpha = 30°$, the DepthError is increased by 15%.

In a particular embodiment of the positioning of the laser handpiece and the depth camera according to the configuration illustrated in Fig. 2B, the angle $\alpha$ between the optical axis of the depth camera and the laser beam is in the range $\alpha = 20°$ to $\alpha = 30°$. Furthermore, the horizontal distance between the depth camera and the laser handpiece is about 45 mm to 55 mm while the vertical distance is about 30 mm to 35 mm. In some embodiments, the camera, particularly the TOF camera, may be positioned at about 5 cm horizontally and about 3 cm vertically relative to the beam exit, approximately parallel, with an angle preferably of about 25°.

Generally, the laser system, particularly the laser handpiece 120, may comprise at least one optical filter such that light and/or radiation incident to the 3D camera is at least partially filtered. In addition, or alternatively, the robotic arm, e.g., the robot system head 134 and/or the robot system end 132, may comprise at least one optical filter such that light and/or radiation incident to the 3D camera is at least partially filtered. In general, 3D camera and/or the optical filters may be configured such that at least a part of the laser light is blocked by the optical filters. Blocking the laser light may comprise that the intensity associated with a certain wavelength of the laser light is attenuated. In some examples, the 3D camera is thus essentially shielded from laser light.

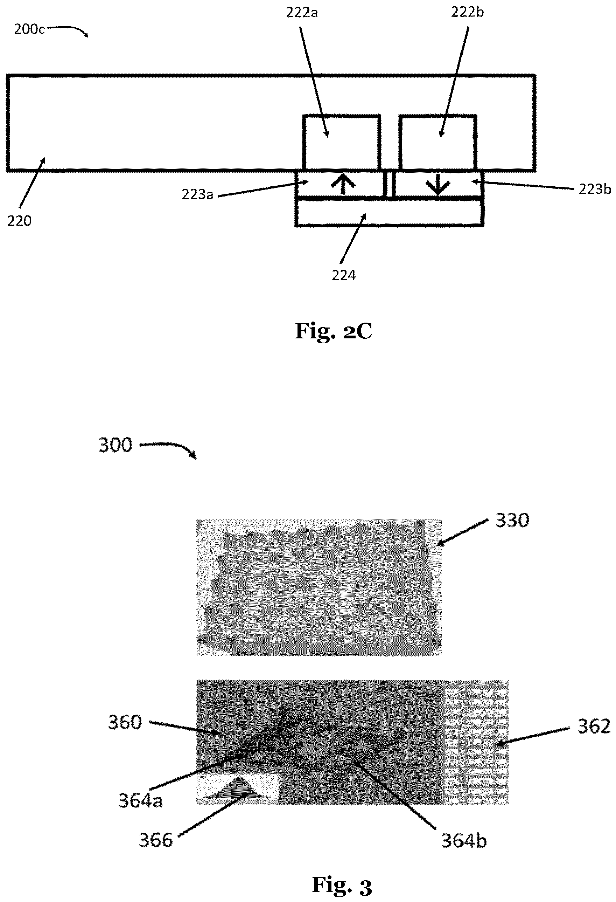

Fig. 2C illustrates a particular arrangement 200c of the 3D camera 200 and optical filters 223a, 223b and 224. The 3D camera 200 comprises a detecting sensor 222a and a light source 222b. For example, the light source 222b may emit radiation and/or light which is scattered by an object and the scattered light may be collected by the detecting sensor 222a. For example, the light source 222b of the 3D camera 200 may emit light with a wavelength in a range of 800 nm to 1000 nm, preferably in a range of 850 nm to 970 nm, most preferably in a range of 900 nm to 950 nm. Specifically, light emitted by light source 222b may first pass through optical filter 223b, then through optical filter 224. Upon scattering from an object, light may return to sensor 222a by passing through optical filter 224 and filter 223a.

Specifically, the optical filter 223a, which may be associated with the detecting sensor 222a, may be a short pass filter. In addition, the optical filter 223b, which may be associated with the light source 222b, may be a short pass filter. The short pass filters 223a, 223b may be associated with a direction (illustrated by the arrows). For example, the short pass filter 223a may be associated with a first orientation and the short pass filter 223b may be associated with a second orientation. In general, the orientation of the short pass filter 223a, 223b may be based on a coating applied to a surface of the short pass filter 223a, 223b and/or an antireflection coating applied to the respective opposite surface. The orientation of the short pass filter 223a, 223b may correspond to a directionality of the short pass filter 223a, 223b. For example, short pass filter 223b may be oriented such that its antireflection coating faces the light source 222b and/or such that its coating faces away from the light source 222b. In some embodiments, the short pass filters 223a, 22b may be arranged such that they have opposite orientations, e.g., they may be flipped by 180°. For example, the short pass filter 223a in front of the detecting sensor 222a may comprise an inward orientation (arrow points towards the 3D camera 200; i.e., coating is applied on that side of the filter 223a) and/or the short pass filter 223b in front of the light source 222b may comprise an outward orientation (arrow points away from the 3D camera 200). In some embodiments, the short pass filter 223a and the short pass filter 223b may be identical, e.g., they may comprise the same physical properties and/or dimensions. For example, the short pass filter 223a, 223b may have a diameter of at least 6 mm, preferably at least 8 mm, more preferably at least 10 mm, most preferably 12 mm and/or a diameter of at most 20 mm, preferably at most 18 mm, more preferably at most 16 mm, most preferably at most 14 mm. Using a short pass filter 223a, 223b comprising an orientation may contribute to a steep decline in the transmission of light by the filter in a vicinity of the respective cutoff wavelength.

Furthermore, the optical filter 224 may be a long pass filter. The long pass filter 224 may not comprise an orientation.

In particular, the cutoff wavelength of the short pass filters 223a, 223b and/or the long pass filter 224 may depend on a wavelength of the light which is emitted by the light source 222b. For example, the short pass filter 223a, 223b may have a first cutoff wavelength and the long pass filter 224 may have a second cutoff wavelength. The first cutoff wavelength and the second cutoff wavelength may be chosen such that a transmitting interval, e.g., a set of wavelengths that can pass through the combination of the short pass filters 223a, 223b and the long pass filter 224, comprises the wavelength of the light which is emitted by the light source 222b. In particular, the lower bound of the transmitting interval may comprise the second cutoff wavelength and/or the upper bound of the transmitting interval may comprise the first cutoff wavelength. Generally, the first and/or second cutoff wavelength and/or the wavelength of the light source 222b may depend on a wavelength of the laser beam. For example, the cutoff wavelength of the short pass filter 223a, 223b may not be larger than 1064 nm. In addition, or alternatively, the cutoff wavelength of the long pass filter 224 may not be smaller than 755 nm. In particular, using a cutoff wavelength of the short pass filter 223a, 223b not larger than 1064 nm may allow to filter laser light generated by a Nd:YAG (1064 nm) laser. Similarly, using a cutoff wavelength of the long pass filter 224 not smaller than 755 nm may allow to filter laser light generated by an Alexandrite (755 nm) laser.

Using optical filters for filtering of the 3D camera light to block the laser light prevents the camera from overheating due to reflected laser light and thus avoids a camera damage.

Generally, the optical filters 223a, 223b, 224 may be arranged in a vicinity of the detecting sensor 222a and/or a light source 222b. For example, the optical filters 223a, 223b, 224 may be arranged such that they are located at a distance from the detecting sensor 222a and/or a light source 222b of at most 15 mm, preferably at most 10 mm, most preferably at most 5 mm.

By arranging the optical filters 223a, 223b, 224 in a vicinity of the detecting sensor 222a and/or a light source 222b the reflection of light from the light source 222b into the detecting sensor 222a due to interaction with the optical filters 223a, 223b, 224 is minimized. In addition, or alternatively, the reflection may be further minimized by increasing a distance between the light source 22b and the detecting sensor 222a, e.g., by disassembling the components of the 3D camera 200.

In some embodiments, moving the laser handpiece 120 and/or the laser beam 125 such that energy is delivered essentially uniformly across the target area may comprise offline procedures, e.g., all procedures which take place before the treatment procedure and/or ii) online procedure and processing, e.g., modules used to perform the treatment procedure in real time.

Generally, offline procedures may comprise a calibration of the camera pose with respect to the robot system head and/or a pre-scanning of the target area and/or target surface and/or a contour registration, and/or path planning. Camera calibration may be performed only once, after mounting, while pre-scanning and registration, which use real-time data processed online, are performed by the physician at the beginning of each treatment.

The offline procedure may comprise a calibration of the camera pose, for example of a depth camera, particularly a 3D TOF camera. In order for the point cloud obtained from the depth camera to best represent the geometry being measured, the camera pose relative to the robot's head may be determined. To this end, multiple point clouds of a reference surface may be acquired from different robot poses and the transformation that aligns them may be determined. Ideally, each point in the point cloud has a position in the world frame that is independent of the current pose of the robot system head. The calibration thus involves the transformation that minimizes the error in the position of the corresponding points in the world frame among point clouds acquired from several different robot poses.

For example, the calibration may depend on the calibration surface and a calibration procedure as well as on a point cloud $\mathbf{P}_{camera}$. The output of the calibration may be used for a pre-scanning, wherein the pre-scanning depends on different poses and a processed point cloud $\mathbf{P}_{processed}$. The pre-scanning may yield a point cloud $\mathbf{P}_{scan}$. The output of the pre-scanning may be used for the registration and the path planning. In particular, the registration may have $\mathbf{P}_{scan}$ as an input and may depend on an input by a physician and may yield a contour C. The path planning may have the contour $\mathbf{C}$, a laser diameter $\mathbf{D}$ as well as $\mathbf{P}_{scan}$ as an input. The path planning may yield a path $\mathbf{p}$ as output.

As illustrated with respect to Figure 3, the calibration 300 may be based on measuring a reference geometry 330 and calculating extrinsic (hand-eye) parameters using numerical optimization of the criteria and/or objective function which represents the error between the point clouds of the reference geometry obtained from different robot poses. A typical result of the calculation is illustrated with respect to the plot 360. The result may comprise hand-eye parameters 362 (3 translations and 3 rotations), the surface 364a, 364b of the reference geometry 330 and a histogram 366 of the deviation between the measured and the reference coordinates along the z axis. In particular, the reference geometry may be measured from different poses 364a, 364b. Here each different measurement pose is illustrated in a different color. For example, 15 different poses may be used.

In some embodiments, the offline procedures may further comprise a pre-scanning, particularly a pre-scanning of at least a part of the target area. A calibrated camera pose may enable the pre-scanning procedure, in which the target surface may be imaged from multiple poses, to obtain a 3D shape of at least a portion of the target area. For example, if the camera is calibrated with sufficient accuracy, the individual point clouds may be merged without major discrepancies. The resulting point cloud $\mathbf{P}_{scan}$ may then serve as a basis for marking the target surface for the treatment, i.e., registering a contour.

Specifically, during the pre-scanning the depth camera may first capture a 3D scan of the patient's body over the maximal robot range area $A_R$, which may be based on a predefined desired maximal range of the robotic arm. This may be accomplished by the robot system positioning and keeping the robot system head perpendicularly to, and at a distance $L_R$ from the (intended) target and/or treatment area, and then sequentially taking 3D images of at least 1 and up to 9 poses.

The distance $L_R$ may depend on the robot range of movement and may be between 400 mm and 1200 mm. Generally, a precision should be better than the dimensions and/or diameters and/or widths of the laser beam, e.g., the spot size $D_x$ and $D_y$.

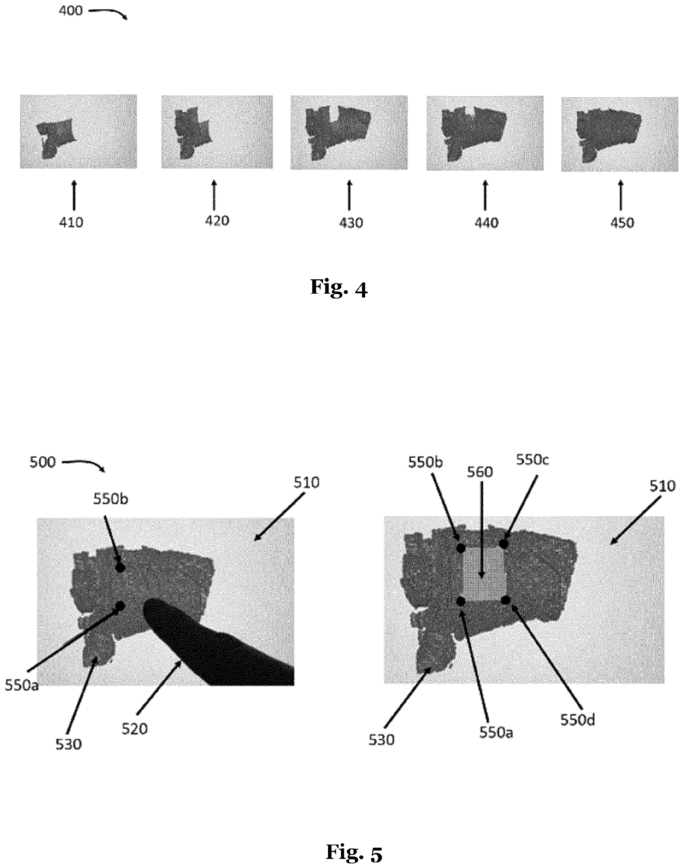

Figure 4 illustrates an exemplary embodiment of a pre-scanning procedure 400. The pre-scanning procedure 400 comprises a pre-scanning procedure during which four corner poses and one central pose of the area $A_R$ are taken. Graphics 410 illustrates a point cloud which is obtained by the camera when positioning the robot system head and/or the camera at a 1st corner of the area $A_R$. In particular, the graphics 410 may be displayed on a user interface. Graphics 420 illustrates a point cloud which is obtained by the camera when positioning the robot system head and/or the camera at a 2nd corner of the area $A_R$. In particular, the graphics 420 may be displayed on a user interface. It can be seen that the area illustrated in graphics 420 is larger than the area illustrated in graphics 410. Similarly, graphics 430 and graphics 440 illustrate a point cloud which is obtained by the camera when positioning the robot system head and/or the camera at a 3rd and 4th corner of the area $A_R$, respectively. Furthermore, graphics 450 illustrate a point cloud which is obtained by the camera when positioning the robot system head at a center of the area $A_R$.

In some embodiments, the offline procedures may further comprise a contour registration. A contour $C_T$ may represent a boundary of the treatment area $A_T$ within the pre-scanned maximal robot range area ($A_R$) of the tissue surface and may be defined by the physician by registering multiple, e.g., at least three, points (e.g., corners) that may define a convex hull of the target (surface) area. The points may be registered by moving the robot system head of the collaborative robot and clicking a physical button and/or by clicking the positions in the point cloud with the mouse on the user interface or with fingers on the user interface, preferably a user interface touch screen.

In particular, with respect to Figure 5, a contour registration by clicking 500 the positions in the point cloud with the mouse on the user interface or with fingers on the user interface is illustrated. In particular, in a first step (left side of Fig. 5), two points 550a and 550b are defined by the user's finger 520 with respect to the pre-scanned maximal robot range area 530 ($A_R$) on the user interface touch screen 510. In a second step (right side of Fig. 5), two further points 550c and 550d may be defined by the user's finger 520 with respect to the pre-scanned maximal robot range area 530 ($A_R$) on the user interface touch screen 510. Based on the four defined points 550a-550d, the target area 560, e.g., the treatment area, may be computed. In particular, the target area 560 may comprise the convex hull of the points 550a-550d.

In some embodiments, the offline procedures may further comprise a path generation. Generally, the goal of the path planning, e.g., path generation, module may comprise to calculate a path of laser beam on the target area with equidistant segments in 3D. This may ensure that the laser irradiation is evenly distributed over the target surface during stamping or brushing. Based on the marked corners and/or points, the robot system's processor may calculate and/or display a robot-generated laser beam path on the selected treatment area $A_T$.

Specifically, the path generation may comprise an algorithm. For example, the algorithm for path planning may require 4 input parameters. In particular, the input parameter may comprise (i) a scan of the patient in the form of a point-cloud; (ii) coordinates of the contour points of the treatment area $C_T$ defined by the clinician; (iii) laser beam spot size $D_x$ and $D_y$ at the working distance from the handpiece, e.g., at the "laser focus"; and/or (iv) a trajectory angle $\beta$.

The algorithm may calculate a series of trajectory points. The basis for the algorithm may comprise the inputs (i) and (ii), namely a scan of the patient's body part and a contour defined by the clinician. In some embodiments, the algorithm may calculate trajectory points starting at the right most edge of the contour, that form a serpentine-like structure moving towards the top edge of the contour, e.g., in the y direction. When the edge in y direction is reached, the algorithm moves in the x direction for the amount of laser width (input (iii)) and continues the serpentine in the -y direction. The process is repeated until the entire contour is covered. The trajectory angle $\beta$ may specify an angle between the trajectory and/or path and the x-axis. In particular, the trajectory angle $\beta$ may specify an inclination between the x-axis and the trajectory. The trajectory angle may depend on the target and/or treatment area and/or its topology. The x-axis may comprise an axis in the x direction. In general, modifying the trajectory angle $\beta$ may enable the user to find a more suitable path for the robot, given the treatment area and its topology.

Generally, for every point in the treatment path, the algorithm may calculate a vector which is normal to the surface defined by the vicinity of the point. The normal may be taken into account when calculating the steps in both x and y directions and it is a vital part of the final output, since the normal defines the angles (pitch, roll, yaw) for the robot at each point. The output of the algorithm may comprise a series of points and their corresponding normal vectors.

In some embodiments, the normal vectors of the generated path may serve as a target orientation of the robot end effector. However, in order to increase the system's working range in terms of reachability, a certain deviation from the normal orientation may be tolerated. The relation between the intensity of fluence of the laser beam and its angle to the normal $\Omega$ may be described by the formula:

$$I = I_0 * \cos \Omega$$

Therein, $I_0$ may be the laser beam intensity measured perpendicular to the beam axis. This tolerance is up to 33°, where the decrease of the laser beam intensity is less than 20% compared to the normal incidence.

Generally, the inclination of the laser beam relative to the surface normal may be denoted by Ω. By allowing a certain amount of inclination, the required working range of the robot can be significantly reduced in comparison to when zero inclination is specified. The robot's working range in case of allowed inclination Ω is reduced:

$$W_{normal} - W_{inclined} = 2 * H * (\sin(\beta) - \sin(\beta - \Omega))$$

where β is an angle of surface normal at the edge of the target area and H is distance between the robot's last rotation axis and the tool center point (**TCP**), i.e., the point and/or position where the robot interacts with the tissue. In particular, the tool center point **TCP** may be a vector, e.g., a vector in three-dimensional space. In other words, the tool center point may be a tool center vector. For example, in case that the system with H = 100 mm needs to process 180° of circumference β = 90°, the $W_{normal}$ - $W_{inclined}$ = 32.3 *mm,* which is approximately 5% of the working range of typical collaborative robot. However, if β = 45°, the $W_{normal}$ - $W_{inclined}$ = 100 mm which is >12% of the typical robot range.

The algorithm for path generation in the robot-assisted laser therapy is intricately designed to handle various challenges posed by the three-dimensional contours of human body parts. The core features of the algorithm outlined above comprise contour point order independence, the handling of concave surfaces, a smoothing the point cloud, a cropping the point cloud, a X-loop and Y-loop as well as ray-casting for in-out determination.

In particular, contour point order independence comprises that the algorithm is designed to be independent of the order of points in the contour. Algorithm orders the points in a way that they represent a closed area.

Furthermore, the handling of concave surfaces comprises that the algorithm's involves processing the point cloud data to manage the intricacies of concave surfaces. If the surface is concave, discontinuities (e.g., islands of points) might remain in the point cloud. The algorithm identifies and removes points whose normal vectors form angles larger than 90 degrees with the global z-axis, to ensure a continuous and smooth path for the laser. This is essential to avoid laser application on inappropriate angles, which could compromise the treatment effectiveness and safety.

Smoothing the point cloud may ensure a more accurate and consistent path, as the algorithm includes a smoothing step. This process refines the point cloud data, reducing noise and irregularities that could affect the trajectory planning.

Cropping the point cloud comprises that the algorithm crops the point cloud to the region of interest defined by the clinician. This step ensures that the path generation focuses solely on the treatment area, enhancing precision and efficiency.

The core of the trajectory planning involves two nested loops - the X-loop and the Y-loop. These loops may iteratively calculate the trajectory points along the x and y directions, respectively, based on the laser beam spot size and the defined treatment area. In some embodiments, a serpentine path may be generated by alternating the direction of the loops at the edges of the treatment area.

The ray-casting for in-out determination comprises to confirm whether each point lies within the designated treatment area, the algorithm employs ray-casting techniques. This step is crucial to ensure that the laser only targets the specified region, avoiding unintended areas.

Specifically, the control algorithm may comprise a point cloud processing and/or a robot' goal pose calculation and/or a control scheme. For example, in the point cloud processing, the first step may comprise cropping the point cloud to a size, which is approximately 2 to 5 times larger than the laser spot size and centered around the spot size. This may be done to calculate accurate distance and orientation of the area, which is actually illuminated by the laser beam. If greater area would be processed, the bigger mistake due to surface uneven curvature would occur. And if smaller area would be cropped, bigger mistake would occur due to measurement noise. In addition, or alternatively, the cropped point cloud may be used to approximate a plane in 3D space using the plane equation:

$$Ax + By + Cz + D = 0.$$

Therein, A, B, C, D are fitting parameters, and x, y, z are coordinates in a global coordinate system, e.g., the robot's space. In addition, or alternatively, an orientation of the robot's end effector and consequently the laser beam is thus determined by the fitted plane's normal (**n**), which is given by **n** = (A, B, C). Further, the distance between the laser handpiece and the surface along the laser beam equals to:

$$dist = \frac{D - \boldsymbol{TCP} * \boldsymbol{n}}{\boldsymbol{k} * \boldsymbol{n}},$$

wherein **TCP** is the "tool working point", which may lie somewhere along the laser beam, e.g., usually in the focus, but not necessary, and **k** is the directional unit vector of the laser beam in global coordinate system. Both parameters (**TCP** and **k**) are available in real-time from robot system and/or the robot controller.

The goal pose calculation, e.g., a calculation of a position **G** and an orientation $q_G$, may determine the input to the robot controller. The aim of the procedure may be to determine the intermediate position of the **TCP** based on the current state described by the laser-surface intersection **N** and/or its normal vector **n** and/or the next target point **P$_i$** defined by the robot path. The target point may be determined in real time based on the point cloud to ensure that the distance is kept constant and the orientation perpendicular to the surface, even if the target surface moves slightly, e.g., due to patient breathing.

The control scheme may be based on a cartesian velocity controller for the robotic system and/or for the robotic arm.

The controller may take the goal position **G** and/or the goal orientation $q_G$ as the input and may obtain the error e by calculating the difference from the current state:

$$e = [\boldsymbol{G} - \boldsymbol{N}, q_G q_N^{-1}].$$

The desired translational and rotational velocities may be calculated separately, as the translational velocity of the end effector, e.g., the laser focus, should be constant, while the target rotations should be followed as closely as possible. The target translational velocity may be obtained by multiplying the target direction **G-N** by a reference velocity $v_{ref}$, subtracting the actual Cartesian velocities v, and using a PI controller to correct the errors. For rotational velocities, the error in orientation, expressed as a quaternion $q_G\, q_N^{-1}$ may be transformed to Euler angles and may be corrected using a separate PI controller. The outputs of the PI controllers may be limited in terms of velocities and/or accelerations and/or jerks to obtain the input for the robot's cartesian velocity controller. When the position of the laser beam on the surface **N** is close enough to the goal **G**, a new goal pose may be calculated based on the next waypoint **P$_{i+1}$**.

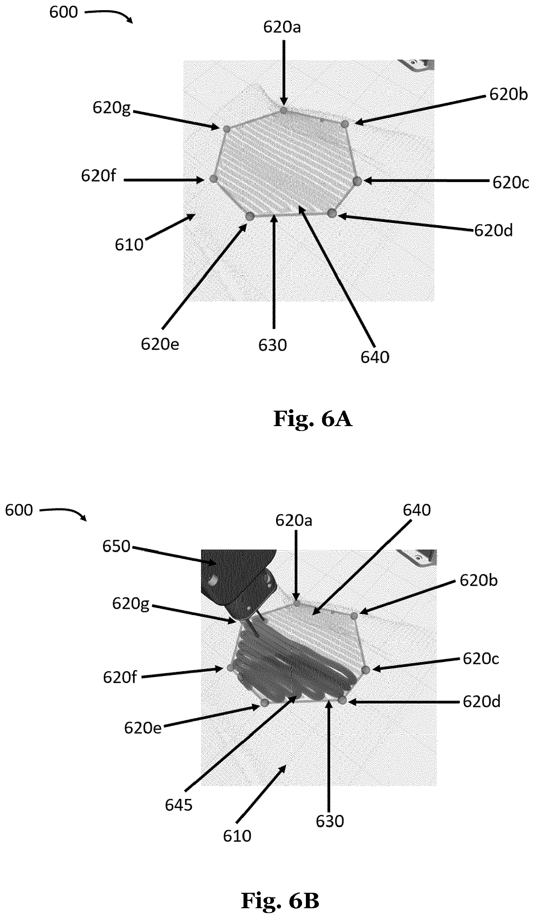

The calculated and/or generated trajectory, e.g., path, is illustrated with respect to Figures 6A and 6B. In particular, the Figs. 6A and 6B illustrate a measured 3D shape of a torso. The measured 3D shape is represented by the measured point cloud 610. Based on the point cloud, the target area 630 may be specified by defining the points 620a-620g. The target area 630 may comprise the convex hull of the defining points 620a-620g. Based on the target area 630, a trajectory 640 may be generated. In particular, Fig. 6A illustrates the trajectory 640 before the robot systems begins to move and before the treatment has started. In addition, Fig. 6B illustrates the results of the achieved trajectory and/or path 645 of the laser spot of the laser handpiece 650 on the target area 630.

In particular, Fig. 6A and Fig. 6B illustrate that the realized device achieves adequate path generation and the corresponding robot guidance. The coverage shown indicates that the achieved paths are suitable for real-world use, as they are much more precise than those achievable by a physician. To validate the proposed method, several tests were performed to verify the ability of the robot to achieve a constant desired laser focus speed at different reference speeds and to maintain a constant distance. Softer settings of the PI controllers may be used, since in therapy it is important that the robot does not perform too jerky movements that cause discomfort or even a rapid motion response from the patient.

In some embodiments, the robotic system and/or apparatus may be configured to perform online procedures. For example, by pressing a "Ready" button, the robot system head may be positioned at a distance $L_T$, which represents the handpiece's working (i.e., laser beam "focus") distance from the treated surface. The range of $L_T$ may be between 50 mm and 500 mm. In addition, or alternatively, by giving a command to the robot system, for example by pressing of the robot system's footswitch, the robot's head's movement is initiated according to the planned laser beam path within the treatment area $A_T$. During this online step, the depth camera may provide feedback in the real-time control loop, i.e., for adaptively controlling, during treatment to compensate for patient's movement, e.g., breathing.

The generated path may represent a guide for the movement of the robot system head and/or the laser handpiece that should result in the desired movement of the laser beam and/or laser spot over the treatment area. However, the actual movement must make sure that the laser beam does not deviate from the surface normal by more than an angle $\Omega$ and from a specified distance at all times, which is made challenging by the possibility of small movements of the patient (e.g., breathing). Therefore, the target pose of the robot system head (i.e., the end effector) may be determined in real time, based on each captured depth camera image, and achieved through a high-frequency control loop. This, online processing, comprises point cloud processing, determination of the goal pose for the laser focus, and control of the robot's head's pose and velocity. The online processing pipeline may thus comprise processing a point cloud $P_{camera}$ by a computer, wherein the processing may comprise cropping. The processing may yield a processed point cloud $P_{processed}$. The normal vectors N, $\mathbf{n}_N$ and a control point cloud $P_{control}$. The control point cloud $P_{control}$, and the normal vectors N, $\mathbf{n}_N$ and a path **p** may be inputs for the goal position calculation. In particular, the goal position calculation may depend on the specific method and may yield as an output the goal **G** and $q_G$. The goal **G** and $q_G$ may be used as inputs for the control, which may depend on the control scheme. The output of the control may comprise a robot state **q.** Generally, the robot state **q**, the normal N, $\mathbf{n}_N$ and the path may be inputs of the laser system. The laser system may further depend on the configuration of an on/off switch.

In some embodiments, the apparatus and/or the robotic laser system may be configured to perform offline procedure. In general, the offline procedure is described for the dual independent robotic laser system configuration since this represents the most complex situation. It will be obvious to someone skilled in the art that for other configurations certain steps can be omitted or simplified (for example, the step 3 in case of the interacting laser and robot systems).

As a first step, the laser handpiece may be installed into the robot system head. In case the robot system may configured for more than one handpiece, one may select the appropriate laser handpiece and/or the laser focus treatment distance $L_T$ on the robot system's user interface. In a second step, appropriate laser parameters on the laser system's user interface for the intended treatment, including any settings for tissue cooling (e.g., DMC cooling) or sensor (e.g., thermal sensor on the laser's handpiece) if applicable, may be selected. In a third step, the selected laser parameters may be input to the robot system via the robot system's user interface. The minimally required laser parameters may comprise the laser spot size ($D_l$), pulse repetition rate (f) and/or the number of pulses ($N_{stack}$) to be delivered to the same spot during a single scan.

In some embodiments, the laser parameters might be read by the robot's depth or color camera. For example, this may be done in a way that robot's camera is oriented towards the laser's monitor, and reads all relevant parameters displayed. Parameters are parsed from the acquired image and displayed on the robot's monitor for user confirmation. In one way, the parameters are parsed as names and numerical values (as for human reading). In addition, or alternatively, the parameters may be coded into bar code or QR code, or into any similar graphical representation.

In a fourth step, based on the input laser parameters, the robot system's processor may calculate a velocity (v) of the laser beam movement in direction x. The velocity (v) of the laser beam movement in direction x may be calculated based on the formula:

$$v = D_x \times f/N_{stack} \cdot$$

It is to be appreciated that with robot-assisted tissue covering the difference between the stamping and brushing technique is only in the value of $N_{stack}$. Thus, when $N_{stack}$ is selected to be equal to 1, the surface coverage will be considered being performed in a stamping manner.

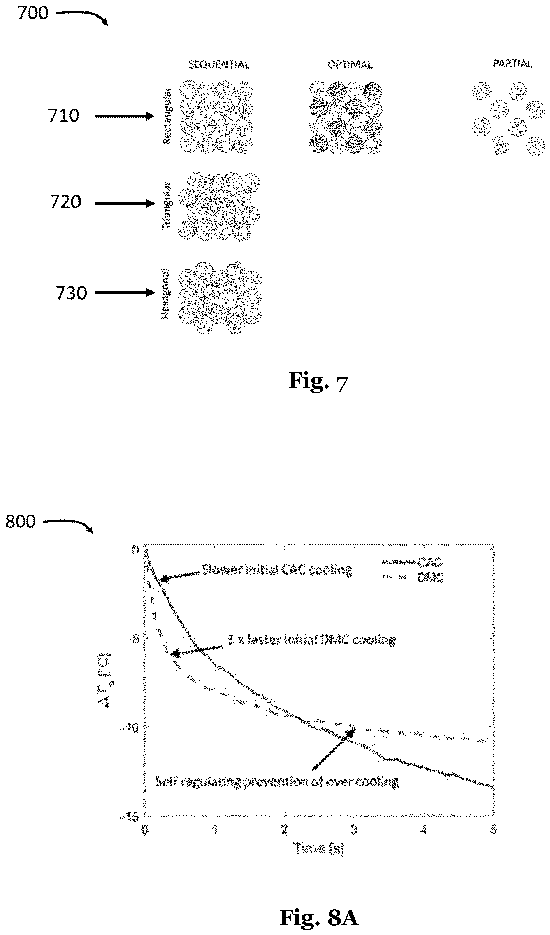

The user may also select on the robot system's user interface the number of coverings ($N_{cov}$), e.g., the number of repeated coverages of the area $A_T$, and as well the desired covering mode. As illustrated in Fig. 7, examples of the covering modes may include the sequential, optimal and partial pattern, wherein for all three modes the spots of the neighboring lines may be positioned in a rectangular 710, triangular 720 or hexagonal pattern 730.

Specifically, the sequential covering mode may perform a line-by-line movement across the entire covering area.

The optimal covering mode may perform the covering across the area $A_T$ in which neighboring, successive spot deposition is avoided. The complete coverage of the area $A_T$ may thus be completed in several passes, e.g., 2 for rectangular and 4 for triangular, across the entire covering area. It is to be noted that for the partial covering mode, $N_{stack} = 0.5$ for rectangular and triangular positioning, and $N_{stack} = 0.67$ for hexagonal positioning.

The partial covering sequence may be based on the same non-successive spot deposition principles as the optimal covering sequence where, however, not all of the passes as required to achieve a complete coverage are performed. The partial covering thus results in a polka-dot patterned coverage.

Generally, the user may also select the desired overlap (for example in % of the laser spot size) in the x ($O_x$) and y ($O_y$) direction. The overlap $O_x$ in the x direction may be selectable only for $N_{stack} = 1$.

In some embodiments, at least one of the steps 2, 3 or 4 may be simplified by defining a group of matching presets for the same treatment, e.g., stamping hair removal, and by selecting the same treatment option or icon on both, the laser and robot system's user interface.

Generally, for many covering procedures, such as, for example during transdermal lipolysis or avalanche mode hair removal, an important treatment parameter may be the cumulatively delivered laser energy per treated area (or the number of laser pulses per treated area, and another parameter per area). In such procedures, the physician may have to estimate the size of the treated area, or simply select the treated body part and rely on an average surface area of that body part.

In this invention, the apparatus and/or robot system may be configured to calculate the area from the marked surface area ($A_T$, and based on the selected treatment type or presets (e.g., lipolysis) and other treatment parameters (e.g., skin type) and laser parameters (e.g., spot size, repetition rate and fluence) calculate and direct the robot to perform the required number of scans which would result in the cumulatively delivered treatment parameter (e.g., energy) as required by the treatment type and other treatment parameters. If the robot movement is programed in a way where the laser beam is inclined to the treated area in order to extend the reachability of the robot as explained above, the system may be configured to adapt the movement speed and/or the step size between the trajectory lines. In this manner the locally delivered cumulative energy per area may be constant regardless of the laser beam inclination, e.g., the angle $\Omega$.

Furthermore, once the body area $A_T$ has been selected, the robot system may be configured to calculate whether the robotic arm is able to perform the covering procedure. Namely, the robot arm may not be able to direct the robot system head and the corresponding laser beam at a certain body part or area which is at an angle not reachable by the arm. Similarly, the required movement of the robot head may require that the robot joints get positioned such that the arm touches or gets blocked by the patient's body.

Some embodiments may comprise online procedure for the apparatus and/or the robotic laser system. The online procedure is described for the dual independent robotic laser system configuration since this represents the most complex situation. It will be obvious to someone skilled in the art that for other configurations certain steps can be omitted or simplified (for example, step 1 in case of the interacting laser and robot systems where a single READY switch might suffice).

As a first step, both, the laser system and the robot system may be switched to the "READY" mode. In a second step, the laser system and the robot system operations may then be simultaneously turned on, for example by pressing the common footswitch. A third step may comprise safety considerations. The footswitch must be under control and for the joint operation pressed by the treating physician. In case any of the systems, either the laser (due to, for example, laser output being detected to be outside of the 20% tolerance) or the robot system (due to, for example, the robot movement being suddenly stopped) detect an error, this may automatically stop the operation of both, regardless of whether the footswitch is pressed. The operation may be automatically stopped also if the system, e.g., the depth sensor, detects rapid and excessive movement of the patient body.

Generally, the apparatus may further comprise means for cooling at least a portion of the target area. In particular, the robotic system, e.g., the robotic arm, may be configured to move the means for cooling across the target area such that cooling is applied essentially uniformly across the target are. For example, the robotic system and/or the robotic arm may be adapted to control, during moving the means for cooling, an effective distance and/or a position and/or an orientation of the means for cooling relative to the target area, preferably at least in part based on the at least one 3D image of the target area.

With laser treatments in dermatology, a non-specific heating of the surface tissue (such as the epidermis) may be a common side effect. Often, the threshold fluence or power which is needed for destroying the target chromophore or structure is very close to the threshold fluence for epidermal injury. For example, in hair removal the optimal laser wavelengths may be the ones which get absorbed in the hair's melanin. Since melanin is abundantly present in the epidermis, heating of the epidermis is an inevitable consequence of the laser light's penetration to the hair bulb to achieve its destruction. Consequently, if the temperature of the epidermis is not controlled, this can in addition to pain induce acute epidermal damage or blistering and can also lead to scarring and hyperpigmentation.

To avoid or minimize the above complications, the epidermal surface layer needs to be cooled. Examples of applications where high-fluence laser pulses are used and cooling is necessary for avoiding epidermal damage include laser hair removal and coagulation of veins and vascular lesions. In other applications, such as transdermal lipolysis, relatively low single-pulse fluences are delivered during a prolonged exposure to repetitively delivered laser pulses. In these applications, epidermal cooling may be applied to prevent a temperature rise of the epidermis while the deeper lying subdermal fat is heated to temperatures above 42°C, which causes apoptosis of adipose cells and subsequent reduction of the fat layer.

There are various methods of skin cooling that are being used during laser treatments, ranging from ice packs to sophisticated equipment. The most frequently used advanced methods include contact cooling with a chilled solid surface, cryogen spray cooling (CSC), and forced cold air cooling (CAC).

Contact cooling with chilled glass achieves localized and rapid cooling but has several disadvantages, including absorption and reflection of the laser light in the chilled glass, skin compression, mandatory use of heat conductive gels, and relatively slow positioning of the small laser spot over the larger treated skin area. A disadvantage of this type of cooling during robot-assisted treatments is that it requires the robot to be in contact with the skin with a controlled contact force. In addition, this type of cooling significantly slows down the procedure.

Cooling with cryogen spray is another commonly used method. In this cooling method, a cryogen spray is sprayed for a very short period only prior to laser pulse delivery, minimizing the exposure of skin to cryogen spray cooled to very low temperatures (down to -58 0C). This method is efficient in epidermal protection when high fluences are used, however, side effects from excessive skin cooling, such as hyperpigmentation and skin irritations have been reported. The method is also very sensitive to the exact timing of the CSC's spurt prior to the laser pulse. Additionally, the pressurized cryogen gas is demanding to transport, and burdensome for the environment, as it has a high global warming potential. It is also expensive since gas cylinders require regular replacements. This cooling method is also not optimal for robot-assisted procedures.

Forced cold air cooling (CAC) is also often used in laser treatments wherein cold air is directed to the treatment area before and during the laser treatment. A disadvantage of air cooling is the relative inefficiency of the medium air for cooling tissues, thus requiring long exposure times to cold air. While CAC cooling is suitable for robot-assisted procedures, it slows down the robot since the robot must move the laser beam across the treated skin surface at sufficiently low speed to establish sufficient skin cooling.

Specifically, the means for cooling may comprise a nozzle for cooling at least a portion of the target area with a fluid. In particular, a method for skin cooling may be the water-based spray cooling described in "Micro-pulsed liquid spray for cooling" patent family US11490945B2, EP3520728 (A1), which is based on dry molecular cooling (DMC) of the skin surface. The advantages of the DMC cooling when used for robot-assisted treatments are that it is non-contact and that the rate of cooling is significantly faster than that of the cold air (CAC) cooling, facilitating much faster movements of the robot across the treated skin area (see figure above). As the laser beam is rapidly swept across the treated surface, and the surface below the cooling fluid output gets cooled only for a short time, the irradiated spots are being cooled down with DMC approximately 3 times faster than with CAC, as illustrated in the diagram 700 in Fig. 8A. For this reason, the DMC cooling is a preferred method for skin cooling during robot-assisted dermatological treatments.

It is to be noted that DMC cooling is already available on commercially available manual and scanning laser handpieces, such as the manual handpiece Fotona R35, or the scanning handpiece Fotona LX Runner. Therefore, by mounting such DMC-equipped handpieces into the robot head, the DMC cooling is readily available for robotic laser systems.

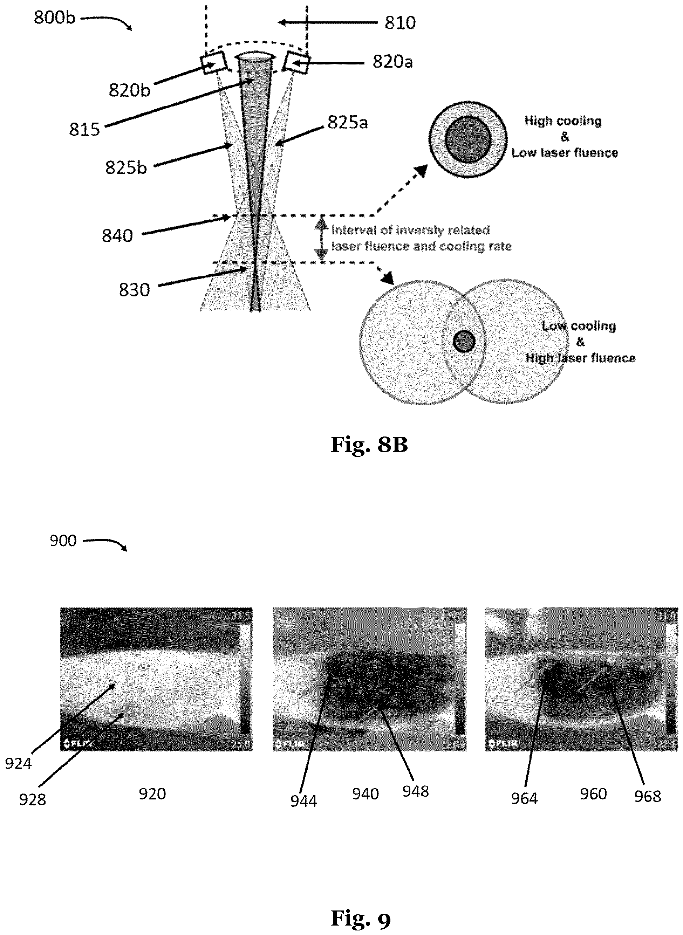

Fig. 8B illustrates the concept of the inverse cooling method 800b. Generally, the inverse cooling method may allow to vary a ratio between the cooling strength applied to a portion of the target area and the energy delivered to a portion of the target area by varying the distance of the laser handpiece and/or the robot end effector to the target area. The apparatus as described herein may be adapted accordingly, and the methods described herein may make use of this property accordingly. In particular, varying the distance may vary a ratio between the cooling strength and a laser irradiation fluence, without changing settings of the cooling and/or laser systems. For example, a laser beam 815 may be emitted by the laser handpiece 810, wherein the laser beam 815 comprises a laser beam focus 830. The laser handpiece may further comprise at least two spray nozzles and/or spray outlets 820a, 820b. The spray nozzles and/or spray outlets 820a, 820b may emit a micro-pulsed liquid spray 825a, 825b, respectively. Generally, the liquid beam of the spray 825a, 825b may be cone-shaped, e.g., has the shape of diverging cones. In some embodiments, the at least two spray nozzles and/or spray outlets 820a, 820b may be arranged at the laser handpiece at a certain distance. In addition, or alternatively, the spray nozzles and/or spray outlets 820a, 820b may be inclined with respect to the laser handpiece 810 and/or the laser beam 815.

In general, depending on the inclination and/or the distance between the spray nozzles and/or spray outlets 820a, 820b, the liquid beams of the spray 825a, 825b may intersect. In particular, there may exist a plane at a distance from the spray nozzles and/or spray outlets 825a, 825b such that a cross-section of the liquid beams of the spray 825a, 825b essentially coincides. This cross-section may be referred to as a cooling spray focus 840. Generally, the distance of the laser focus 830 and the distance of the cooling spray focus 840 to the laser handpiece 810 may be different. For example, the distance of the laser focus 830 to the laser handpiece 810 may be larger than the distance of the cooling spray focus 840. In addition, or alternatively, a width of the laser beam focus 830 may be different from a width of the cooling spray focus 840. For example, the width of the cooling spray focus 840 may be larger than the width of the laser beam focus.

In general, the spot size of the laser beam 815 on the target area and the spot size of the liquid beams 825a, 825b on the target area may depend on a distance of the laser handpiece 810 and/or a distance of the spray nozzles and/or the spray outlets 820a, 820b from the target area. In particular, the laser irradiation fluence and/or irradiation dosage of the laser beam 815 may be changed by changing the distance of the laser handpiece to the target area. Similarly, the cooling strength of the cooling spray may be changed by changing the distance of the spray nozzles and/or spray outlets 820a, 820b to the target area.

Positioning the laser beam focus 830 below the cooling spray focus 840 may result in an interval of inversely related irradiation and cooling. In the plane of cooling spray focus 840, the cooling may be most intense and the beam fluence may be lower than compared to the plane of laser beam focus 830, where beam fluence may be at its maximum. On the other hand, in the plane of the laser beam focus 830, the cooling may be reduced due to increased area. By adaptively changing the working distance, the apparatus and/or the robot may dynamically change the ratio between irradiation and cooling power in real time. Effectively, the irradiation dosage could be regulated by robot movement only, without controlling the laser and/or the cooling system. In particular, this may be used for adapting the energy input in real time without communicating to the laser system and/or the means for cooling. Therefore, the inverse cooling method 800b is a good solution for keeping the robotic system and the laser system and/or the cooling system separate and/or independent. Generally, the inverse cooling method 800b may for example be used in the context of the dual, independent system.

In some embodiments, the treatment of the target area may comprise a selection of exclusion areas. The selection of exclusion areas to may allow to avoid sensitive skin areas such as heavy pigmentation, scars, etc. The selection of exclusion areas to may be performed using the grayscale image captured by the depth camera and/or the color image captured by the color camera in the visual light spectrum and/or the image captured by the IR camera. Based on this information, the algorithm may recognize the areas that should be excluded from the treatment. The system can either automatically exclude the detected areas or leave the final decision to the clinician. In addition, the algorithm may allow the clinician to interactively change the exclusion areas, e.g., by adding, removing, widening or shrinking of the sensitive areas, on the graphical display. The exclusion areas may represent an additional input for the path generation algorithm described above.

In some embodiments, the apparatus and/or the robotic laser system may comprise a detection of laser pulses and synchronization. When the noninteracting robotic laser system configuration is used, the robot system has no information on the timing of laser pulses communicated directly from the laser system. Since in certain embodiments it is desirable that the robot head movement is synchronized with the laser pulse emissions this can be accomplished by installing on the laser head a detector of laser light reflected from the irradiated tissue surface.

In some embodiments, the apparatus and/or the robotic laser system may comprise a scanning handpiece plus a robotic scanning. In certain embodiments, the speed v (given by $v = D_x \times f/N_{stack}$) of the robot-assisted movement of the perpendicularly delivered beam spot across the treated surface may be limited due to the performance limitations of the robotic system or due to the patient not being comfortable with the rapid movements of the robot's arm above the patient's body. This may limit the achievable coverage rate R representing the area covered by laser irradiation in certain time (in $cm^2/s$), and the maximal laser repetition rate ($f_{max}$) to $R_{max} = S \times f_{max}$ (for $N_{stack} = 1$). Since the laser pulse fluence F (in $J/cm^2$) and the laser pulse energy E (in J) may be limited by the maximally acceptable $F_{max}$ resulting from the requirement that the tissue should not overheat during a pulse, the limited coverage rate limits the average laser power P (in W) which can be delivered during the robot-assisted treatment to $P_{max} = F_{max} \times R_{max} = E_{max} \times f_{max}$. This may lead to the under-utilization of the available laser power and longer duration of laser treatments than necessary.

Thus, in an alternative embodiment, the fast beam movements may be performed by a scanning handpiece. In those embodiments, the robotic arm would provide the slower movements, for example for repositioning of the scanning handpiece from one scanning segment to another, or for the slow brushing of the continuously scanned segment across the tissue surface. The fast movements of the beam would in such embodiments be realized by the scanning handpiece being capable of delivering higher repetition rates than $f_{max}$. In one of the preferred embodiments, the scanning handpiece parameters, e.g., scan size, shape, overlap etc., may be set on the user interface of the laser system, and controlled by the laser system. It is to be appreciated that the scanning by the scanning handpiece may be performed in different modalities (e.g., a sequential, optimal, partial, rectangular, triangular, hexagonal) and spot overlaps, scan sizes and shapes, e.g., linear, square, triangular etc.

For example, in one of the embodiments, the scanning handpiece may generate a rapidly scanned line of laser spots, and the robot may cover the larger area $A_T$ by moving the scanned line with dimension $D_x$ across the treatment surface at a slower speed. In yet another embodiment, the scanning handpiece may scan the beam over a certain area $A_s$, with the dimension $D_{sx}$ in the direction of the robot's movement and $D_{sy}$ in the direction perpendicular to the direction of the robot's movement, with a single scan lasting for the time $t_s$. In such an embodiment, the robot system may calculate, correspondingly the speed of movement from $v = D_{sx} \times (1/t_s)/N_{stack}$, where $N_{stack}$ in this embodiment represents the number of repeated irradiations of the area $A_s$. Any overlaps $O_{sx}$ and $O_{sy}$ may then be defined, for example as % of dimensions $D_{sx}$ and $D_{sy}$.

In addition, or alternatively, the rapid beam movements may be generated by the robot itself. In these embodiments, the rapid movements are made possible by loosening the requirement that the laser beam is perpendicular to the surface. In this case the rapid beam movements may be produced by the rapid movement of the ending robot arm joints in order for the beam direction to deviate from the perpendicular direction within the angle of $\pm 33°$, where the intensity decreases for less than 20%.

In general, the adaptive control of the robotic arm and/or the laser system and/or the means for cooling may be based on a temperature profile of the at least portion of the target area to which energy is delivered. For example, the adaptive control may comprise a control of the laser energy deposition in the tissue by monitoring the temporal evolution of the temperature of the irradiated skin. The monitoring of the temporal evolution may be achieved by using a temperature camera, e.g., an infra-red camera. The temperature camera may be integrated in the laser end effector and/or laser handpiece, see for instance the temperature camera 190 in Fig. 1A or Fig. 1B. The laser end effector and/or laser handpiece may be attached to the robotic arm. The deposition of the laser energy may be controlled by adjusting the laser power (in the case of CW operation) and/or the laser energy per pulse and the repetition rate (in the case of pulsed operation) and/or the speed of movement of the laser beam along the generated path and/or a diameter of the laser beam (in the case of CW or pulsed operation).

In particular, the laser power and/or laser energy per pulse and/or the repetition rate and/or or the speed of movement of the laser beam along the generated path and/or a diameter of the laser beam may be used as an input for the control, particularly for a control of the robotic arm and/or the laser system and/or the laser handpiece and/or the means for cooling.

In some embodiments, the control may be based on a current temperature of the illuminated skin, e.g., where the laser beam illuminates the skin. In addition, or alternatively, the control may be based on a current temperature of the skin area to be illuminated in the next short time. For example, in CW mode, this may mean 0.1 to 2 beam diameters before the current position of the laser beam. In pulsed mode, this may mean 1 to 10 laser pulses before the current position of the laser beam.

In addition, or alternatively, the control may be based on a heating and/or cooling rate e.g., an increase and/or decrease in temperature over time, of the illuminated area and its surroundings that have recently been illuminated. With this information, the temperature-depth profile may be estimated and may be used to control the heating of the subcutaneous tissue.

It is to be noted that matrix temperature sensors (MatrixView®) are already available on commercially available manual and scanning laser handpieces, such as the manual handpiece Fotona R35, or the scanning handpiece Fotona LX Runner. Therefore, by mounting such Fotona MatrixView-equipped handpieces into the robot system head, the temperature sensing which is controlling the laser system operation is readily available to be used with robotic laser systems.

In some embodiments, the adaptive control may comprise a patient in-plane movement detection. For example, the patient in-plane movement detection may be based on thermal imaging. Generally, it may be that a depth camera can only detect an out-of-plane movement of the target area, e.g., breathing of the patient. As explained above, the out-of-plane movement may be used as feedback to control the motion and/or movement of the robotic arm. However, the depth camera may not detect when at least a portion of the target area moves in-plane, e.g., perpendicular to the viewing direction. In general, this movement must be detected in order to adjust the movement, e.g., to adaptively control the movement, of the robot system and/or the robotic arm and thus accurately transfer the laser energy of the laser beam to the target area.

This can be achieved in an embodiment, where the apparatus comprises an IR camera not only to control the laser energy delivery in the tissue, but also to detect possible in-plane movement of the target area. As illustrated with respect to Figure 9, this may be achieved by capturing thermal images 900 of the target area and recognizing representative features 924, 928, 944, 948, 964, 968 on the respective image 920, 940, 960, which may then be used to track its movement in-plane over time. In general, tracking may be done based on various known algorithms, e.g., digital image correlation, optical flow and/or feature detection.

As can best be seen in Fig. 9, when using thermal imaging, e.g., an IR camera, the thermal field of the skin 920, 940, 960 within the target area may provide more than enough useful features 924, 928, 944, 948, 964, 968 to track them during the treatment and/or during processing. In particular, the thermal field 920 of the untreated skin already has enough features 924, 928 to track. Even better contrast may be achieved by cooling, e.g., the thermal field 940 providing the features 944, 948. The cooling may comprise water spray cooling. Similarly, a better contrast may be achieved by cooling and laser illumination, e.g., the thermal field 960 providing the features 964, 968. In addition, or alternatively, a better contrast may be achieved by laser illumination only. Generally, the features may comprise high-contrast features which can be used for tracking of in-plane movement of the target area.

Therefore, a complete 3D movement of the target area may be measured and/or tracked. For example, complete 3D movement of the target area may be measured and/or tracked by merging measurements of with IR camera, as it is described above, and of depth camera.

The advantage of this solution compared to using a colour camera is that no additional markings (such as stickers or drawings) are required on the skin. Consequently, the process is cleaner and undisturbed. When using a colour camera, the markers must be positioned outside the target area. For this reason, the viewing area of the colour camera should be larger than the target area so that the camera cannot be positioned on the end effector of the robotic system and/or robotic arm. The colour camera must therefore be positioned in a separate, e.g., distant, location, which complicates the system considerably.

In some embodiments, the treatment of the target area may comprise a self-calibration of the apparatus. In particular, self-calibration may be extremely important to ensure accurate execution of the pre-programmed therapy within optimal and safe limits.

For example, the self-calibration may comprise determining a geometry configuration between the robot system, e.g., the robotic arm, the laser end effector and integrated sensors, e.g., TOF camera, visual camera and/or IR camera, in such a way that the actual position of the laser beam on the skin surface matches the one used for path generation. This part of the self-calibration may be done by measuring the shape of the reference body, which is known as etalon, and then calculating the geometry parameters so that the deviation between the measured and reference shape is minimized.

In addition, or alternatively, the self-calibration may comprise a determination of a thermal response of the patient's tissue to the laser energy deposition. In this part of the self-calibration, the physical parameters such as light absorptivity and/or thermal conductivity and/or heat capacity and/or density, etc. may be calculated based on the measurement of the change in temperature over time during and/or after laser illumination of a small portion of a representative skin area. The laser energy may be selected at a low level to avoid overexposure.

**Claims**

1. An apparatus for treatment of a target area of a patient by an energy beam, comprising:

means for receiving at least one 3D image of the target area;

means for delivering the energy beam to at least a portion of the target area;

means for automatically moving the energy beam, preferably by moving at least a portion of the means for delivering the energy beam, such that energy is delivered essentially uniformly across the target area;

wherein the apparatus is adapted to control, during the moving of the energy beam, an effective distance of the means for delivering the energy beam and/or an orientation of the energy beam relative to the target area, at least in part based on the at least one 3D image of the target area.

2. The apparatus according to claim 1, wherein:

the control of the effective distance comprises a control of a spatial distance to the target area and/or a control of a focus of the means for delivering the energy beam; and/or

the control of the orientation comprises a control of an orientation of at least a portion of the means for delivering the energy beam relative to the at least portion of the target area.

3. The apparatus according to claim 1 or 2, wherein the apparatus is further configured to, during the moving of the energy beam:

maintain an essentially perpendicular orientation relative to the target area.

4. The apparatus according to one of the claims 1 to 3, further comprising means for cooling at least a portion of the target area, wherein the apparatus is preferably configured to automatically move the means for cooling across the target area such that cooling is applied essentially uniformly across the target area.

5. The apparatus according to claim 4, wherein:

the apparatus is adapted to control, during moving the means for cooling, an effective distance of the means for cooling and/or an orientation of the means for cooling relative to the target area, preferably at least in part based on the at least one 3D image of the target area.

6. The apparatus according to one of the claims 1 to 5, wherein the means for automatically moving is configured to move the means for delivering the energy beam based on a parameter of the energy beam and/or a parameter of the cooling and/or a target treatment profile.

7. The apparatus according to claim 6, wherein the parameter of the cooling is at least in part based on the parameter of the energy beam and/or the target treatment profile.

8. The apparatus according to one of the claims 1 to 7, wherein the means for receiving the at least one 3D image comprises a camera, preferably a depth-sensing camera, and/or is at least partially attached to the means for delivering the energy beam and/or the means for automatically moving.

9. The apparatus according to claim 8, wherein:

the camera is attached such that its optical axis essentially forms a right angle with respect to the energy beam; and

further comprising a dichromatic mirror, wherein the dichromatic mirror is arranged such as to transmit the laser beam and to at least partially reflect radiation to which the camera is sensitive to.

10. The apparatus according to one of the claims 1 to 9, further configured to adaptively control, while moving the energy beam, a position relative to at least a portion of the target area to which the energy beam is delivered, the effective distance and/or the orientation of the energy beam relative to at least a portion of the target area, wherein the adaptive control is at least in part based on an image of at least a portion of the target acquired while moving the energy beam.

11. The apparatus according to claim 10, wherein:

the adaptive control is at least in part based on a height of the at least a portion of the target area to which the energy beam is delivered;

preferably wherein the height is based on an image from a height sensor of the apparatus and/or from the means for receiving the 3D image; and/or

the adaptive control is at least in part based on a temperature profile of the at least a portion of the target area to which the energy beam is delivered.

12. The apparatus according to one of the claims 1 to 11, further comprising means for determining a temperature of at least a portion of the target area, preferably wherein the means for determining a temperature comprise an infrared camera.

13. The apparatus according to one of the claims 1 to 12, wherein the means for automatically moving comprises at least one robotic arm; preferably wherein the robotic arm is movable with respect to at least 3 degrees of freedom.

14. The apparatus according to one of the claims 1 to 13, wherein the energy beam comprises a laser beam; preferably wherein the laser beam is generated by a Nd:YAG laser and/or an Alexandrite laser and/or an Er:YAG laser and/or a diode laser and/or a $CO_2$ laser.

15. Apparatus according to one of the claims 1 to 14, further comprising a footswitch and/or a handheld control-device, wherein the footswitch and/or the handheld control-device is configured to at least partially control the means for delivering the energy beam and/or the means for cooling and/or the means for automatically moving.

16. The apparatus according to one of the claims 1 to 15, further comprising at least one user interface for controlling the means for delivering the energy beam and/or the means for cooling and/or the means for automatically moving; preferably wherein the at least one user interface is configured to receive a user input identifying the target area within a graphical representation of at least a portion of the patient; wherein the graphical representation is based on the at least one 3D image of the target area.

17. A control method, comprising:

receiving at least one 3D image of a target area of a patient for treatment by an energy beam;
determining a configuration according to which to move the energy beam relative to the target area based on the at least one 3D image;
wherein the configuration comprises an effective distance of means for delivering the energy beam and/or an orientation of the energy beam relative to the target area;
moving the energy beam across the target area based on the determined configuration, such that energy is delivered essentially uniformly across the target area.

18. A computer program comprising instructions for performing the method according to claim 17 when the instructions are executed.

**Fig. 1A**

**Fig. 1B**

200

230

210

220

225

215

240

**Fig. 2A**

200

210

α

220

215

225

240

**Fig. 2B**

200c

222a

222b

223a

223b

220

224

**Fig. 2C**

300

330

360

362

364a

366

364b

**Fig. 3**

**Fig. 4**

**Fig. 5**

**Fig. 6A**

**Fig. 6B**

700

710 →

720 →

730 →

**Fig. 7**

800

**Fig. 8A**

**Fig. 8B**

**Fig. 9**

Europäisches
Patentamt

European
Patent Office

Office européen
des brevets

# EUROPEAN SEARCH REPORT

**Application Number**

EP 24 17 8004

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/145514 A1 (GROSSMAN DAN DOV [IL] ET AL) 20 May 2021 (2021-05-20) | 1-4,6, 8-16 | INV. A61B18/203 |
| A | * paragraphs [0094] - [0101], [0181], [0186], [0198], [0200], [0221] - [0224]; figures 2,18,20B, 25 * | 5,7 | |
| X | WO 2023/023866 A1 (LIGHT MATTER INTERACTION INC [CA]) 2 March 2023 (2023-03-02) | 1-3,6, 8-16 | |
| A | * figures 14A,14B * | 4,5,7 | |
| X | CN 111 920 446 A (ZHANG JIMIN; LI TONG) 13 November 2020 (2020-11-13) | 1-3,6,8, 10,12, 13,15,16 | |
| A | * claims 9-11; figures 1A,3 * | 4,5,7,9, 11,14 | |
| X | US 2019/105200 A1 (HIPSLEY ANNMARIE [US]) 11 April 2019 (2019-04-11) | 1,4,6, 8-11, 13-16 | |
| A | * paragraphs [0292], [0331], [0339], [0360], [0437], [0477], [0478], [0514], [0531], [0567]; figures 3B,4A,6,7 * | 5,12 | TECHNICAL FIELDS SEARCHED (IPC) A61B A61F A61N A61C |
| X | US 2008/033410 A1 (RASTEGAR JAHANGIR S [US] ET AL) 7 February 2008 (2008-02-07) | 1-3,6, 8-16 | |
| A | * paragraphs [0013], [0014], [0026], [0060], [0077] - [0079], [0082], [0099], [0103], [0118]; figures 3,4,7 * | 4,5,7 | |
| X | US 2021/128236 A1 (CODD PATRICK [US] ET AL) 6 May 2021 (2021-05-06) | 1-3,6, 9-16 | |
| A | * paragraphs [0001] - [0015], [0030], [0033], [0042], [0062] - [0065], [0079]; figure 1 * | 4,5,7,8 | |

-/--

The present search report has been drawn up for all claims

| Place of search | Date of completion of the search | Examiner |
|---|---|---|
| Munich | 11 September 2024 | Link, Tatiana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 17 8004

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 112 717 281 A (CHONGQING HANHENG MEDICAL TECH CO LTD) 30 April 2021 (2021-04-30) | 1-4,6, 8-16 | |
| A | * the whole document * | 5,7 | |
| X | CN 109 259 822 B (UNIV XI AN JIAOTONG) 24 December 2019 (2019-12-24) | 1-3,6, 8-10,12, 13,15,16 | |
| A | * claims 1-6,9,10; figure 1 * | 4,5,7, 11,14 | |
| X | US 2015/209599 A1 (UNIV LELAND STANFORD JUNIOR [US]) 30 July 2015 (2015-07-30) * paragraphs [0013], [0097], [0106]; figure 1 * | 1-3,6, 10,16 | |
| | | | **TECHNICAL FIELDS SEARCHED (IPC)** |

The present search report has been drawn up for all claims

| Place of search | Date of completion of the search | Examiner |
|---|---|---|
| Munich | 11 September 2024 | Link, Tatiana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                                   

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 8004

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2024

| Patent document cited in search report | Publication date | Patent family member(s) | Publication date |
|---|---|---|---|
| US 2021145514 A1 | 20-05-2021 | AU 2019294577 A1 | 28-01-2021 |
| | | BR 112020026654 A2 | 23-03-2021 |
| | | CA 3104963 A1 | 02-01-2020 |
| | | CN 112423689 A | 26-02-2021 |
| | | EP 3813708 A1 | 05-05-2021 |
| | | GB 2589798 A | 09-06-2021 |
| | | IL 279784 A | 01-03-2021 |
| | | JP 2021529613 A | 04-11-2021 |
| | | JP 2024102335 A | 30-07-2024 |
| | | KR 20210024603 A | 05-03-2021 |
| | | US 2021145514 A1 | 20-05-2021 |
| | | US 2023031007 A1 | 02-02-2023 |
| | | WO 2020003138 A1 | 02-01-2020 |
| WO 2023023866 A1 | 02-03-2023 | CA 3171496 A1 | 27-02-2023 |
| | | EP 4391944 A1 | 03-07-2024 |
| | | KR 20240076781 A | 30-05-2024 |
| | | WO 2023023866 A1 | 02-03-2023 |
| CN 111920446 A | 13-11-2020 | NONE | |
| US 2019105200 A1 | 11-04-2019 | AU 2018243837 A1 | 21-11-2019 |
| | | AU 2024200128 A1 | 25-01-2024 |
| | | BR 112019020532 A2 | 28-04-2020 |
| | | CA 3058461 A1 | 04-10-2018 |
| | | CN 110891511 A | 17-03-2020 |
| | | CN 116712252 A | 08-09-2023 |
| | | EP 3600109 A1 | 05-02-2020 |
| | | EP 4438016 A2 | 02-10-2024 |
| | | ES 2969893 T3 | 23-05-2024 |
| | | IL 269740 A | 28-11-2019 |
| | | JP 2020512917 A | 30-04-2020 |
| | | JP 2023164422 A | 10-11-2023 |
| | | KR 20200024132 A | 06-03-2020 |
| | | KR 20230154479 A | 08-11-2023 |
| | | RU 2019134801 A | 30-04-2021 |
| | | RU 2022103876 A | 29-03-2022 |
| | | SG 11201909096W A | 30-10-2019 |
| | | TW 201941750 A | 01-11-2019 |
| | | US 2019105200 A1 | 11-04-2019 |
| | | US 2021338484 A1 | 04-11-2021 |
| | | WO 2018183987 A1 | 04-10-2018 |
| US 2008033410 A1 | 07-02-2008 | US 2008033410 A1 | 07-02-2008 |
| | | US 2010087803 A1 | 08-04-2010 |
| | | US 2010094265 A1 | 15-04-2010 |

EPO FORM P0459

For more details about this annex : see Official Journal of the European Patent Office, No. 12/82

page 1 of 2

**EP 4 652 948 A1**

## ANNEX TO THE EUROPEAN SEARCH REPORT
## ON EUROPEAN PATENT APPLICATION NO.

EP 24 17 8004

11-09-2024

| Patent document cited in search report | Publication date | Patent family member(s) | Publication date |
|---|---|---|---|
| US 2021128236 A1 | 06-05-2021 | US 2021128236 A1<br>WO 2018152538 A1 | 06-05-2021<br>23-08-2018 |
| CN 112717281 A | 30-04-2021 | NONE | |
| CN 109259822 B | 24-12-2019 | NONE | |
| US 2015209599 A1 | 30-07-2015 | CN 104869912 A<br>EP 2877097 A1<br>US 2015209599 A1<br>WO 2014018983 A1 | 26-08-2015<br>03-06-2015<br>30-07-2015<br>30-01-2014 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6273885 B1
- US 20070208326 A1
- EP 3520728 A1
- EP 4098217 A1
- EP 22211909 A
- EP 23174965
- US 11490945 B2